(19) Europäisches Patentamt
European Patent Office
Office européen des brevets

(11) Publication number: 0 159 180 B1

(12) **EUROPEAN PATENT SPECIFICATION**

(45) Date of publication of patent specification: 09.08.89

(51) Int. Cl.[4]: C 07 K 9/00, A 61 K 37/02, C 12 P 21/04 // (C12P21/04, C12R1:365)

(21) Application number: 85302544.3

(22) Date of filing: 11.04.85

(54) **Improvements in and relating to antibiotics M43A, M43B, M43C and M43D.**

(30) Priority: 16.04.84 US 600725
16.04.84 US 600726
16.04.84 US 600728
16.04.84 US 600729

(43) Date of publication of application:
23.10.85 Bulletin 85/43

(45) Publication of the grant of the patent:
09.08.89 Bulletin 89/32

(84) Designated Contracting States:
AT BE CH DE FR GB IT LI NL SE

(58) References cited:
EP-A-0 112 184
US-A-3 067 099
JOURNAL OF THE AMERICAN CHEMICAL SOCIETY, vol. 104, no. 15, 28th July 1982, pages 4293-4295, American Chemical Society, US; C.M. HARRIS et al.: "Structure of the glycopeptide antibiotic vancomycin. Evidence for an asparagine residue in the peptide"

(73) Proprietor: ELI LILLY AND COMPANY
Lilly Corporate Center
Indianapolis Indiana 46285 (US)

(72) Inventor: Higgins, Harvey M., Jr.
R.R. 3, Box 337
Danville Indiana 46122 (US)
Inventor: McCormick, Mack Harold
2361 22nd Street North Court
Florence Oregon 97439 (US)
Inventor: Merkel, Kurt Edgar
11608 Mann Road
Mooresville Indiana 46158 (US)
Inventor: Michel, Karl Heinz
222 E. North Street Apt. 2205
Indianapolis Indiana 46204 (US)

(74) Representative: Hudson, Christopher Mark et al
Erl Wood Manor
Windlesham Surrey GU20 6PH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 159 180 B1

**Description**

The present invention concerns novel antibiotic derivatives of vancomycin, designated M43A, M43B, M43C and M43D. The derivatives are products of the fermentation of *N. orientalis* M5—18260 (NRRL 2452) and *N. orientalis* M43—05865 (NRRL 2450).

New, improved antibiotics are continually in demand, particularly for the treatment of human diseases. Increased potency, expanded spectrum of bacterial inhibition, increased *in vivo* efficacy, and improved pharmaceutical properties (such as greater oral absorption, higher blood or tissue concentrations, longer *in vivo* half life, and more advantageous rate or route of excretion and rate or pattern of metabolism) are some of the goals for improved antibiotics.

In the search for new antibiotics, structural modification of known antibiotics is attempted whenever possible. This approach is limited, however, to modifications which retain the desired activity. Many antibiotics, including the glycopeptides, have such complex structures that even small changes can be difficult to make by chemical means. The discovery of new antibiotics produced by fermentation processes continues, therefore, to be of great importance even in cases where the antibiotic, once recognized, is quite similar to a previously known antibiotic.

Antibiotics M43A, M43B, M43C and M43D are newly discovered members of the glycopeptide group of antibiotics. A closely related member of this group includes vancomycin (see, for example, U.S. Patent 3,067,099). In U.S. Patent 3,067,099, McCormick *et al.* described the preparation of vancomycin. Three strains of *Streptomyces orientalis*, two of which were numbered M43—05865 and M5—18260, were disclosed as being capable of making vancomycin. The cultures were deposited at what was then the Northern Regional Research Laboratories (now known as the Midwest Area Northern Regional Research Center) at Peoria, Illinois, given the accession numbers NRRL 2450 (M43—05865) and NRRL 2452 (M5—18260). Later, the organism designation for the strains was changed from *Streptomyces orientalis* to *Nocardia orientalis*.

The vancomycin described in U.S. 3,067,099 became an important, commercially available antibiotic. The *N. orientalis* culture used to prepare the commercial product was *N. orientalis* strain M5—18260 (NRRL 2452) or its progeny. *N. orientalis* M43—05865 (NRRL 2450) strain produces both antibiotic M43A and vancomycin as major factors.

The structure of a closely related derivative of vancomycin was determined by Sheldrick *et al.* [G. M. Sheldrick, P. G. Jones, O. Kennard, D. H. Williams and G. A. Smith, *Nature 271* (5642), 223—225 (1978)]; later, the structure of vancomycin itself was found by Harris *et al.* [C. M. Harris and T. M. Harris, *J. Am. Chem. Soc. 104*, 4293—4295 (1982)] to be that shown in formula *1*:

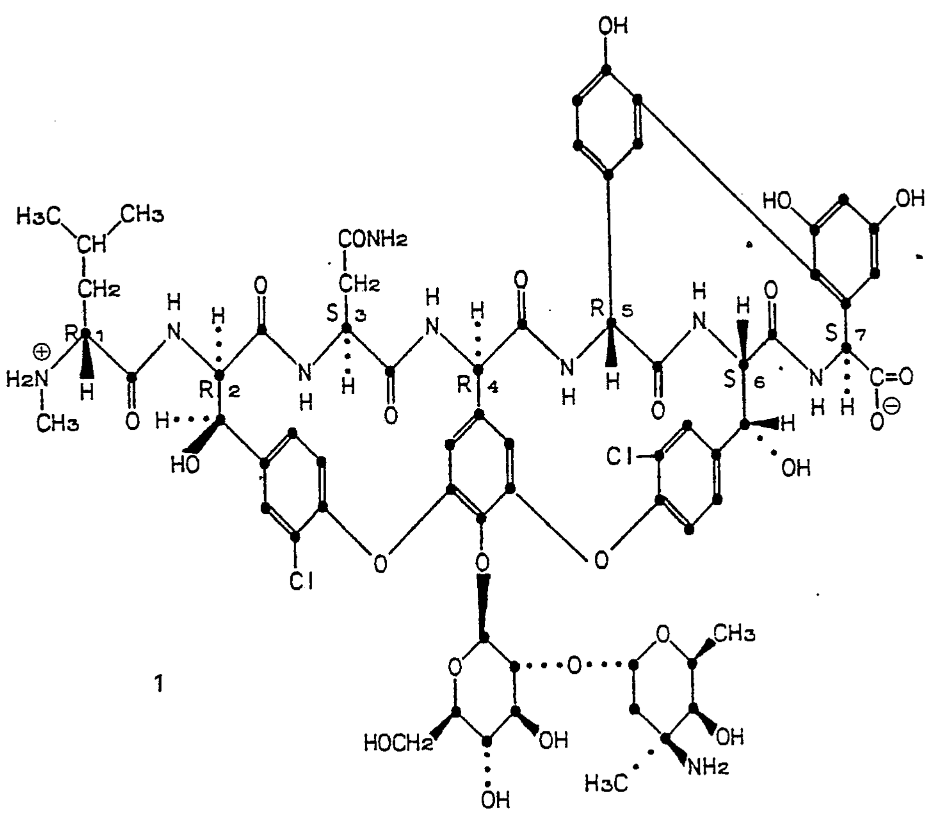

1

This invention concerns antibiotic M5—18260 or M43—05865 complex, or M43A, M43B, M43C or M43D, or a pharmaceutically-acceptable salt thereof, associated with less than 10% by weight of vancomycin.

This invention also concerns new antibiotic complex called M43A, M43B, M43C and M43D which have the formula *2*:

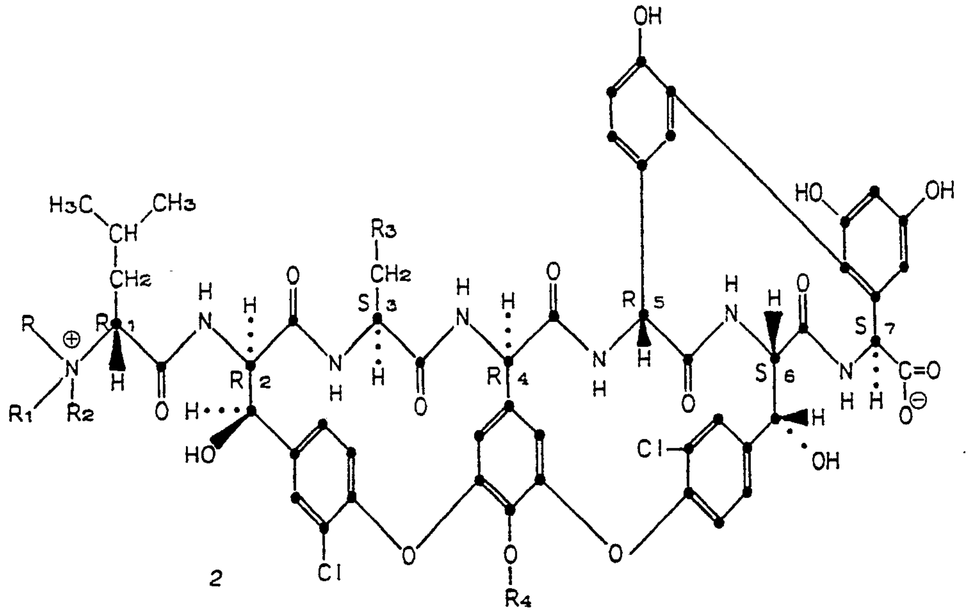

2

wherein:

R is H or methyl;
$R_1$ and $R_2$ are methyl;
$R_3$ is $CONH_2$ or COOH;
$R_4$ is glucosyl or vancosaminyl-O-glucosyl;
provided that when $R_4$ is glucosyl, then R must be methyl and $R_3$ must be $CONH_2$; or a pharmaceutically-acceptable salt thereof; and
associated with less than 10% by weight of vancomycin.

M43A has structural formula *3*:

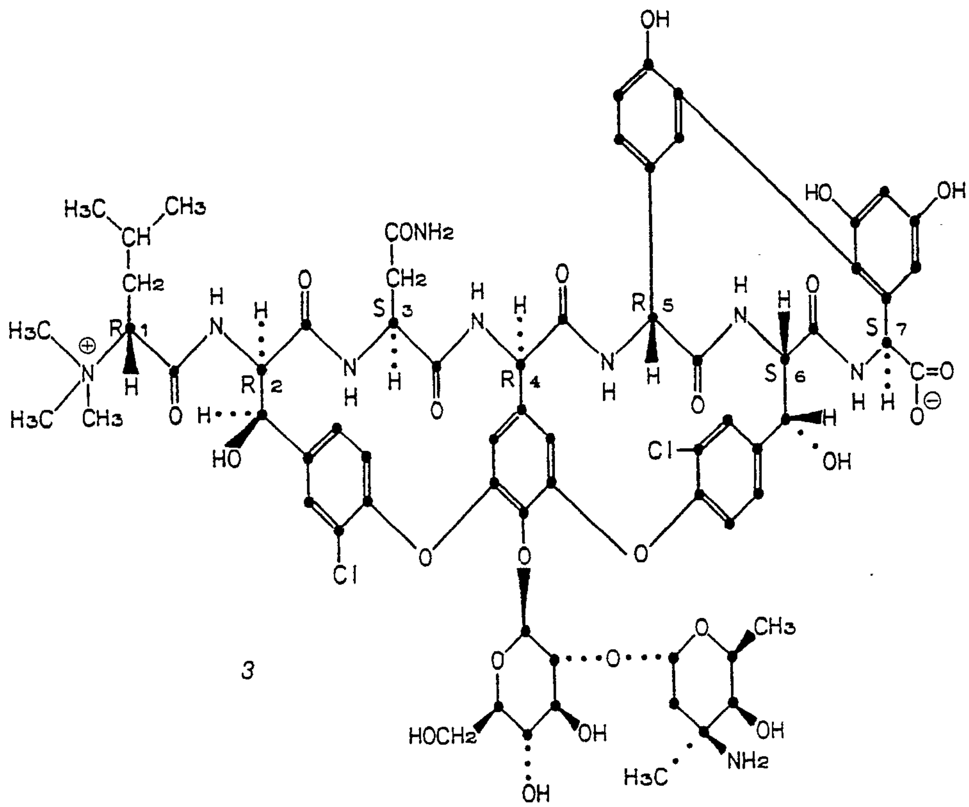

3

or a pharmaceutically-acceptable salt thereof, associated with less than 10% by weight of vancomycin. M43A is described in formula *2* when R, $R_1$ and $R_2$ are methyl, $R_3$ is $CONH_2$, and $R_4$ is vancosaminyl-O-glucosyl.

M43B has structural formula *4*:

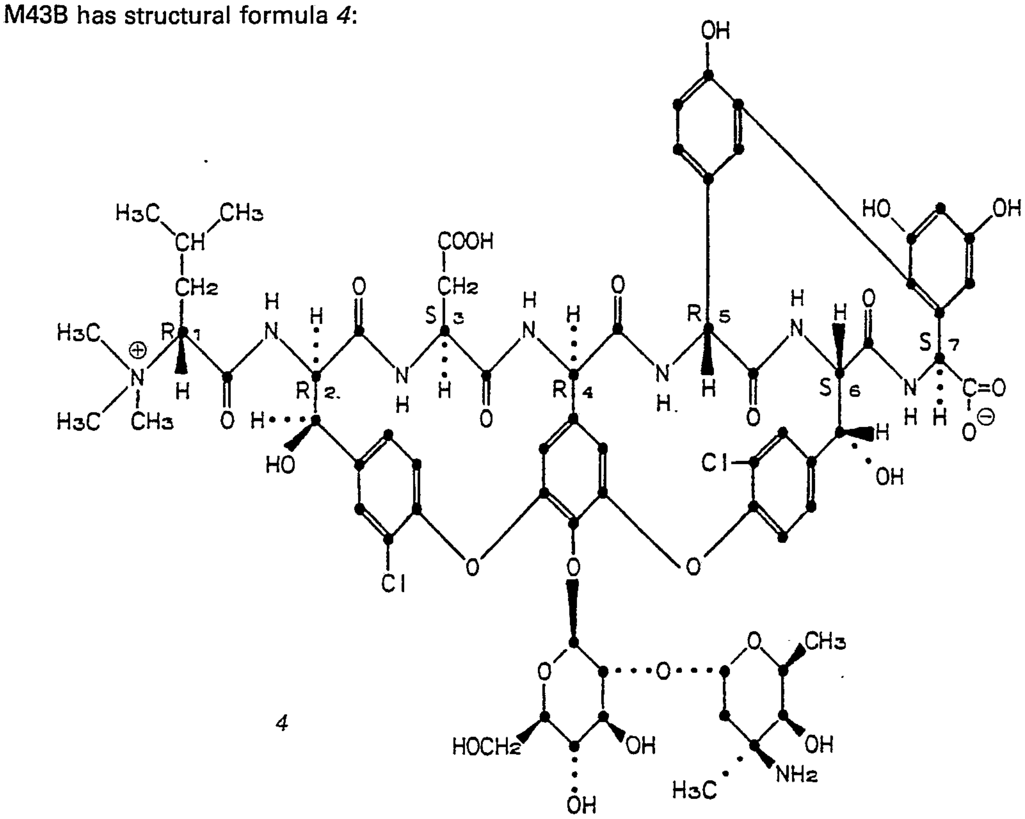

4

or a pharmaceutically-acceptable salt thereof, associated with less than 10% by weight of vancomycin. M43B is described in formula *2* when R, $R_1$ and $R_2$ are methyl, $R_3$ is COOH, and $R_4$ is vancosaminyl-O-glucosyl.

M43C has structural formula *5*:

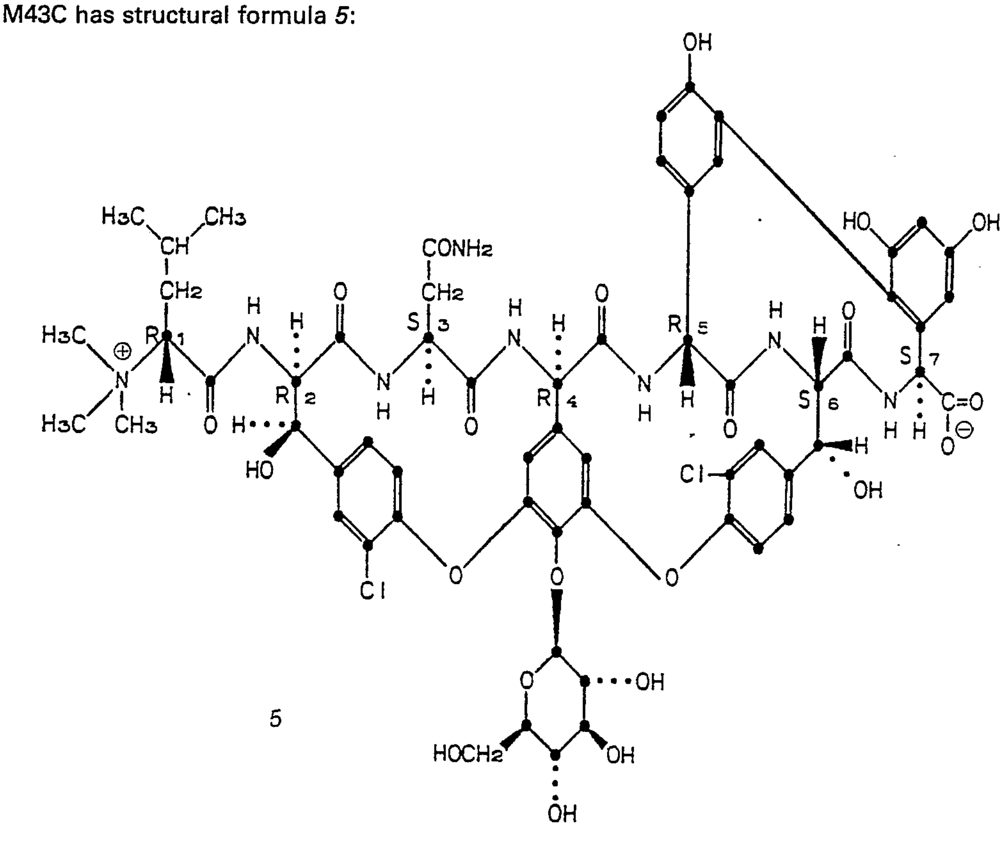

5

or a pharmaceutically-acceptable salt thereof, associated with less than 10% by weight of vancomycin. M43C is described in formula *2* when R, $R_1$ and $R_2$ are methyl, $R_3$ is $CONH_2$, and $R_4$ is glucosyl.

M43D has structural formula *6*:

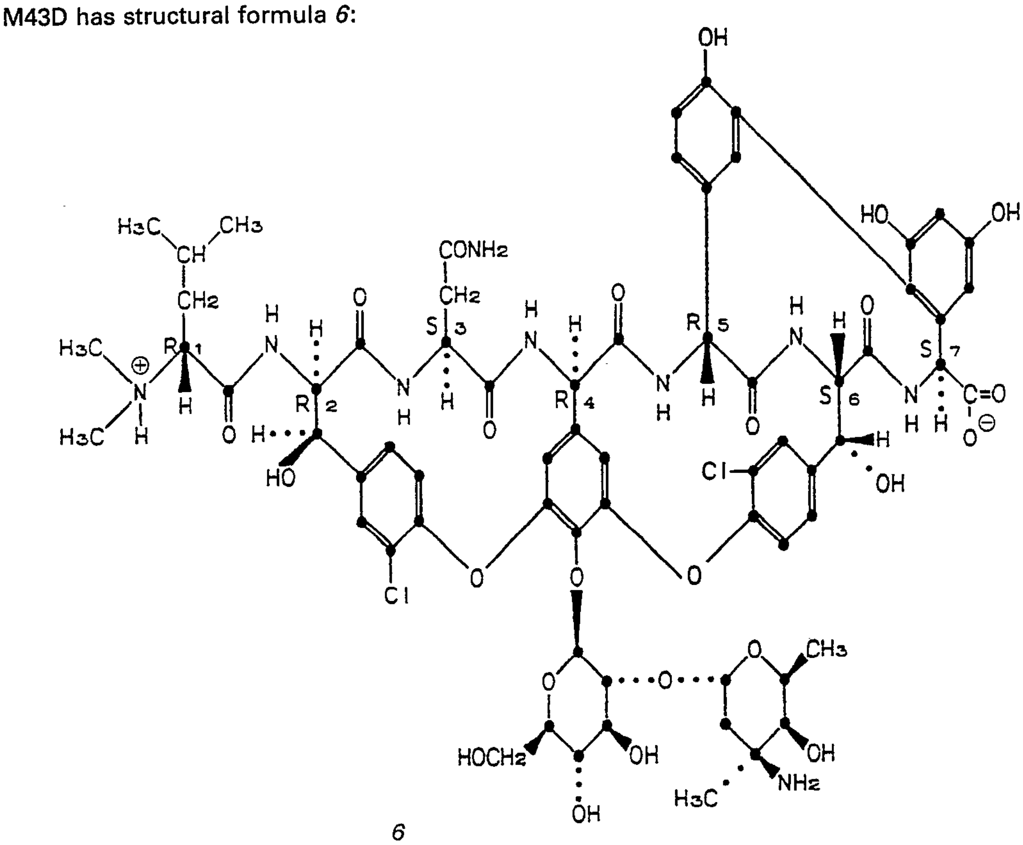

*6*

or a pharmaceutically-acceptable salt thereof, associated with less than 10% by weight of vancomycin. M43D is described in formula *2* when R is H, $R_1$ and $R_2$ are methyl, $R_3$ is $CONH_2$, and $R_4$ is vancosaminyl-O-glucosyl.

For convenience, the terms 'vancosaminyl-O-glucosyl' and 'glucosyl' will be used herein to denote α-O-vancosaminyl-β-O-glucosyl and β-O-glucosyl, respectively. Also, the terms 'M43 complex' or 'antibiotic(s)' refer to M43A, M43B, M43C and/or M43D.

Antibiotics M43A, M43B, M43C, and M43D have excellent antibacterial activity. The pharmaceutically-acceptable salts of the derivatives of formula *2* are especially useful.

The *N. orientalis* M5—18260 (NRRL 2452) strain produces very small amounts of M43A, M43B and M43C. In addition to vancomycin, the antibiotic complex produced by *N. orientalis* M43—05865 (NRRL 2450), also called the M43 antibiotic complex, contains antibiotic M43A as a major factor. The ratio of M43A to vancomycin produced by the M43—05865 (NRRL 2450) strain is approximately 2.5 to 1. In addition, the M43 complex contains a number of minor factors. Among the minor M43A factors are 1) A51568 factor A and A51568 factor B; 2) antibiotic M43D; 3) the compounds designated agluco-A51568A, aglucovancomycin and agluco-M43A, and desvancosamine-vancomycin; and 4) antibiotic M43C and antibiotic M43B. The structural relationships of this group of antibiotics are provided in formula *2* when designated as follows:

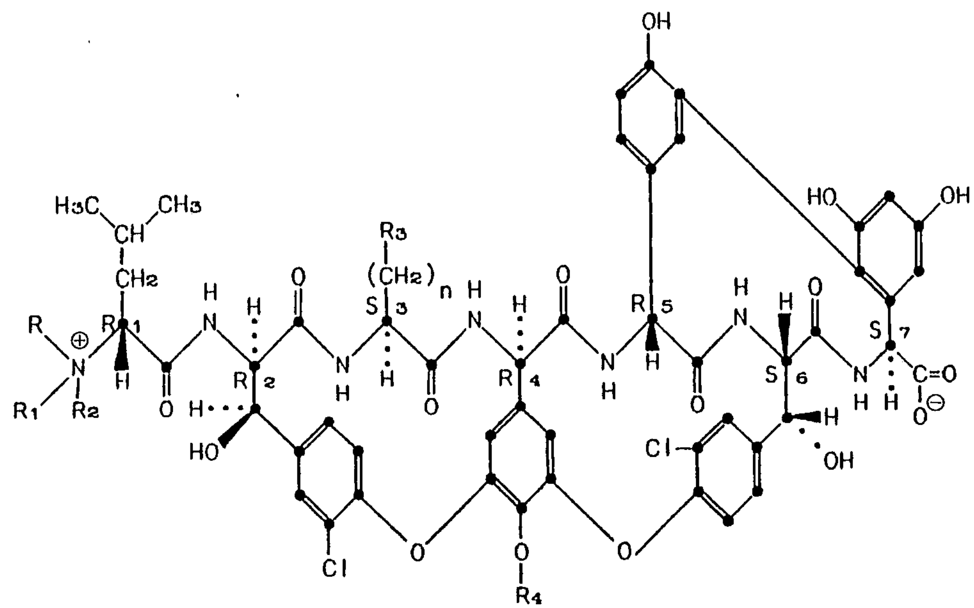

2

| Compound No. | Compound | R | $R_1$ | $R_2$ | $R_3$ | n | $R_4$ |
|---|---|---|---|---|---|---|---|
| 1 | Vancomycin | H | H | $CH_3$ | $CONH_2$ | 1 | vancosaminyl-O-glucosyl |
| 2 | M43A | $CH_3$ | $CH_3$ | $CH_3$ | $CONH_2$ | 1 | " " |
| 3 | M43B | $CH_3$ | $CH_3$ | $CH_3$ | COOH | 1 | " " |
| 4 | M43C | $CH_3$ | $CH_3$ | $CH_3$ | $CONH_2$ | 1 | glucosyl |
| 5 | M43D | H | $CH_3$ | $CH_3$ | $CONH_2$ | 1 | vancosaminyl-O-glucosyl |
| 6 | Agluco-A51568A | H | H | H | $CONH_2$ | 1 | H |
| 7 | Aglucovancomycin | H | H | $CH_3$ | $CONH_2$ | 1 | H |
| 8 | Agluco-M43A | $CH_3$ | $CH_3$ | $CH_3$ | $CONH_2$ | 1 | H |
| 9 | Desvancosamine-A51568A | H | H | H | $CONH_2$ | 1 | glucosyl |
| 10 | Desvancosamine-Vancomycin | H | H | $CH_3$ | $CONH_2$ | 1 | glucosyl |
| 11 | A51568A | H | H | H | $CONH_2$ | 1 | vancosaminyl-O-glucosyl |
| 12 | A51568B | H | H | H | $CONH_2$ | 2 | " " |

M43A, M43B, M43C and M43D, the new glycopeptide antibiotics of formula *2*, are close to vancomycin in structure and also in activity. They are, therefore, valuable additions to this group of antibiotics.

M43A, M43B, M43C and M43D are shown in formula *2* as zwitterions. Those skilled in the art will recognize, however, that each has one or two carboxyl groups, one or two amino groups and three phenolic groups which can react to form various salts. All such forms of M43A, M43B, M43C and M43D are part of this invention. The salts are useful, for example, for separating and purifying the antibiotics. In addition, the salts have an improved solubility in water.

M43A, M43B, M43C and M43D salts are prepared using standard procedures for salt preparation. For example, the formula *2* zwitterion can be neutralized with an appropriate acid to form an acid addition salt.

The acid addition salts of formula *2* are particularly useful. Representative suitable salts include those salts formed by standard reactions with both organic and inorganic acids such as, for example, sulfuric, hydrochloric, phosphoric, acetic, succinic, citric, lactic, maleic, fumaric, cholic, pamoic, mucic, D-glutamic, *d*-camphoric, glutaric, glycolic, phthalic, tartaric, formic, lauric, stearic, salicylic, methanesulfonic, benzenesulfonic, sorbic, picric, benzoic, cinnamic and like acids.

Pharmaceutically acceptable acid addition salts are an especially preferred group of salts of formula *2*.

Antibiotics M43A, M43B, M43C and M43D are prepared by culturing *Nocardia orientalis* NRRL 2450 or antibiotics M43A, M43B and M43C are prepared by culturing *N. orientalis* NRRL 2452, or an M43A, M43B, M43C or M43D-producing variant, mutant or recombinant thereof, under submerged aerobic conditions in a suitable culture medium until a substantial amount of M43A, M43B, M43C or M43D is produced. Also a method of producing the M5—18260 or M43—05865 antibiotic complex or M43A, M43B, M43C or M43D as hereinbefore defined comprises cultivating *N. orientalis* NRRL 2450 or NRRL 2452, or an M5—18260 or M43—05865 producing varient, mutant or recombinant thereof, in a culture medium containing assimilable sources of carbon, nitrogen, and inorganic salts under submerged aerobic fermentation conditions. M43A, M43B, M43C or M43D is then separated from the complex. The culture medium used to grow *Nocardia orientalis* NRRL 2450 or NRRL 2452 can be one of a number of media. For economy in production, optimal yield, and ease of product isolation, however, certain culture media are preferred. Thus, for example, preferred carbon sources include carbohydrates such as dextrin, dextrose, glucose and glycerol. Preferred nitrogen sources include enzyme digests of casein, cottonseed meal, soybean grits, protein peptones and the like. Among the nutrient inorganic salts which can be incorporated in the culture media are the customary soluble salts capable of yielding iron, potassium, sodium, magnesium, calcium, ammonium, chloride, carbonate, sulfate, nitrate, and like ions.

Essential trace elements necessary for the growth and development of the organism should also be included in the culture medium. Such trace elements commonly occur as impurities in other constituents of the medium in amounts sufficient to meet the growth requirements of the organism. It may be necessary to add small amounts (i.e. 0.2 ml/L) of an antifoam agent such as polypropylene glycol (M.W. about 2000) to large-scale fermentation media if foaming becomes a problem.

For production of substantial quantities of antibiotics M43A, M43B, M43C and M43D, submerged aerobic fermentation in tanks is preferred. Small quantities may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form of mycelial fragments of the organism to obtain a fresh, actively growing culture of the organism. The vegetative inoculum is then transferred to a larger tank. The medium used for the vegetative inoculum can be the same as that used for larger fermentations, but other media can also be used.

*N. orientalis* NRRL 2450 or NRRL 2452 can be grown at temperatures between about 25° and about 37°C. Optimum antibiotic production appears to occur at temperatures of about 30°C.

As is customary in aerobic submerged culture processes, sterile air is bubbled through the culture medium. For efficient antibiotic production the percent of air saturation for tank production should be about 50% or above [at 30°C and about 5 psi ($0.34 \times 10^6$ dynes/cm$^2$) of back pressure].

Antibiotic production can be followed during the fermentation by testing samples of the broth against organisms known to be sensitive to the antibiotics. One useful assay organism is *Staphylococcus aureus* NRRL B313. In addition, antibiotic production can be monitored by HPLC with UV detection.

Following its production under submerged aerobic fermentation conditions, the antibiotics can be recovered from the fermentation medium by filtering the broth to remove mycelia and purifying the filtered broth by a series of adsorptions on suitable adsorbents, such as an ion-exchange resins, chemically modified hydrophobic inorganic supports used in high performance reversed-phase liquid chromatography, and high porosity polymers, eluting the antibiotics in each case with a suitable solvent such as aqueous acetonitrile.

M43A, M43B, M43C and M43D inhibit the growth of a broad spectrum of pathogenic bacteria, especially gram-positive bacteria. Table I summarizes the minimal inhibitory concentrations (MIC's) at which the antibiotics inhibit certain organisms, as determined by standard agar-dilution assays. In Table I the activity of M43A and M43D (each as the phosphate salt) and M43B and M43C (free base) are compared with that of vancomycin (free base).

TABLE I

In Vitro Activity of M43A, M43B, M43C and M43D

| Organism | MIC (mcg/ml) | |
|---|---|---|
| | Vancomycin | M43A |
| *Staphylococcus aureus* NRRL B313 | 1 | 1 |
| *Staphylococcus aureus* V41 | 1 | 1 |
| *Staphylococcus aureus* X400 | 1 | 2 |
| *Staphylococcus aureus* S13E | 1 | 1 |
| *Staphylococcus epidermidis* EPI1 | 2 | 4 |
| *Staphylococcus epidermidis* 222 | 1 | 2 |
| *Streptococcus pyogenes* C203 | 1 | 1 |
| *Streptococcus pneumoniae* Park 1 | 0.5 | 0.5 |
| *Streptococcus faecium* ATCC 9790 | 1 | 1 |
| *Streptococcus sp.* group D 9960 | 4 | 4 |
| *Haemophilus influenzae* C.L. | 128 | 64 |
| *Haemophilus influenzae* 76 | 32 | 64 |
| *Escherichia coli* N10 | >128 | >64 |
| *Escherichia coli* EC14 | >128 | >64 |
| *Escherichia coli* TEM | >128 | >64 |
| *Klebsiella pneumoniae* X26 | >128 | >64 |
| *Klebsiella pneumoniae* X68 | >128 | >64 |
| *Klebsiella pneumoniae* KAE | >128 | >64 |

TABLE I (continued)

In Vitro Activity of M43B and M43C

| Organism | MIC (mcg/ml) M43B | M43C | Vancomycin |
|---|---|---|---|
| *Staphylococcus aureus* NRRL B313 | 16 | 2 | 1 |
| *Staphylococcus aureus* V41 | 32 | 4 | 1 |
| *Staphylococcus aureus* X400 | 32 | 4 | 2 |
| *Staphylococcus aureus* S13E | 32 | 4 | 1 |
| *Staphylococcus epidermidis* EPI1 | 32 | 8 | 4 |
| *Staphylococcus epidermidis* 222 | 32 | 8 | 2 |
| *Streptococcus pyogenes* C203 | 16 | 2 | 1 |
| *Streptococcus pneumoniae* Park 1 | 2 | 2 | 0.25 |
| *Streptococcus faecium* ATCC 9790 | 32 | 4 | 2 |
| *Streptococcus sp.* group D 2041 | 32 | 8 | 4 |
| *Haemophilus influenzae* C.L. | >64 | >64 | 64 |
| *Haemophilus influenzae* 76 | >64 | 32 | 16 |
| *Escherichia coli* N10 | >64 | >64 | >128 |
| *Escherichia coli* EC14 | >64 | >64 | >128 |
| *Escherichia coli* TEM | >64 | >64 | >128 |
| *Klebsiella pneumoniae* X26 | >64 | >64 | >128 |
| *Klebsiella pneumoniae* X68 | >64 | >64 | >128 |
| *Klebsiella pneumoniae* KAE | >64 | >64 | >128 |

TABLE I (continued)

In Vitro Activity of M43D

| Organism | MIC (mcg/ml) Vancomycin | M43D |
|---|---|---|
| *Staphylococcus aureus* NRRL B313 | 0.5 | 1 |
| *Staphylococcus aureus* V41 | 0.5 | 1 |
| *Staphylococcus aureus* X400 | 1 | 2 |
| *Staphylococcus aureus* S13E | 1 | 1 |
| *Staphylococcus epidermidis* EPI1 | 2 | 4 |
| *Staphylococcus epidermidis* 222 | 1 | 2 |
| *Streptococcus pyogenes* C203 | 0.5 | 0.5 |
| *Streptococcus pneumoniae* Park 1 | 0.125 | 0.25 |
| *Streptococcus faecium* ATCC 9790 | 1 | 2 |
| *Streptococcus sp.* group D 2041 | 4 | 4 |
| *Haemophilus influenzae* C.L. | 64 | >64 |
| *Haemophilis influenzae* 76 | 128 | >64 |
| *Escherichia coli* N10 | >128 | >64 |
| *Escherichia coli* EC14 | >128 | >64 |
| *Escherichia coli* TEM | 64 | >64 |
| *Klebsiella pneumoniae* X26 | >128 | >64 |
| *Klebsiella pneumoniae* X68 | >128 | >64 |
| *Klebsiella pneumoniae* KAE | >128 | >64 |

M43A also inhibits the growth of anaerobic bacteria. Table II summarizes the susceptibility of various anaerobic isolates to M43A.

TABLE II

Susceptibility of Anaerobic Bacterial Isolates to M43A

| Anaerobic Bacteria | MIC (μg/ml)[a] Vancomycin | M43A |
|---|---|---|
| *Clostridium difficile* 2994 | 2 | 1 |
| *Clostridium perfringens* 81 | 1 | 1 |
| *Clostridium septicum* 1128 | 4 | 32 |
| *Eubacterium aerofaciens* 1235 | 16 | 4 |
| *Peptococcus asaccharolyticus* 1302 | 2 | 2 |
| *Peptococcus prevoti* 1281 | 2 | 1 |
| *Peptostreptococcus anaerobius* 1428 | 2 | 0.5 |
| *Peptostreptococcus intermedius* 1264 | 4 | 8 |
| *Propionibacterium acnes* 79 | 2 | 1 |
| *Bacteroides fragilis* 111 | 64 | 8 |
| *Bacteroides fragilis* 1877 | 32 | ≤0.125 |
| *Bacteroides fragilis* 1936B | 32 | 2 |
| *Bacteroides thetaiotaomicron* 1438 | 16 | 8 |
| *Bacteroides melaninogenicus* 1856/28 | >128 | ≤0.125 |
| *Bacteroides melaninogenicus* 2736 | 8 | ≤0.125 |
| *Bacteroides vulgatis* 1211 | 16 | ≤0.125 |
| *Bacteroides corrodens* 1874 | 32 | ≤0.125 |
| *Fusobacterium symbiosum* 1470 | 2 | ≥0.125 |
| *Fusobacterium necrophorum* 6054A | 2 | 2 |

[a]MIC's were determined by the agar-dilution method; endpoints were read after 24-hrs. incubation.

M43A and M43C have also shown *in vivo* antimicrobial activity against experimental bacterial infections. When two doses of test compound were administered to mice in experimental infections, the activity observed was measured as an $ED_{50}$ value [effective dose in mg/kg to protect 50% of the test animals: see Warren Wick, *et al., J. Bacteriol. 81*, 233—235 (1961)]. $ED_{50}$ values observed for M43A and M43C are given in Table III.

TABLE III

$ED_{50}$ Values for M43A and M43C

| Organism | Route of Administration | $ED_{50}$ (mg/kg/2) | | |
|---|---|---|---|---|
| | | Vancomycin | M43A | M43C |
| *Staphylococcus aureus* | subcutaneous | 1.62 | 0.5 | — |
| *Streptococcus pyogenes* | " | 1.2 | 1.36 | — |
| *Streptococus pneumoniae* | " | 1.47 | 0.88 | 2.67[a] |

[a]LD Challenge Dose = 125

Pharmaceutical formulations which comprise as active ingredient M43A, M43B, M43C or M43D or a pharmaceutically-acceptable salt thereof (an M43 complex), associated with less than 10% by weight of vancomycin and associated with one or more pharmaceutically-acceptable carriers or diluents therefor, are also part of this invention. The antibiotic, preferably as a pharmaceutically-acceptable salt, can be formulated for oral or parenteral administration for the therapeutic or prophylactic treatment of bacterial infections. For example, an M43 complex can be admixed with conventional pharmaceutical carriers and excipients and used in the form of tablets, capsules, elixirs, suspensions, syrups, wafers and the like. The compositions comprising an M43 complex will contain from about 0.1 to about 90% by weight of the active compound, and more generally from about 10 to about 30%. The compositions may contain common carriers and excipients, such as corn starch or gelatin, lactose, sucrose, microcrystalline cellulose, kaolin, mannitol, dicalcium phosphate, sodium chloride and alginic acid. Disintegrators commonly used in the formulations of this invention include croscarmellose sodium, microcrystalline cellulose, corn starch, sodium starch glycolate and alginic acid. Tablet binders that can be included are acacia, methylcellulose, sodium carboxymethylcellulose, polyvinylpyrrolidone (Povidone), hydroxypropyl methylcellulose, sucrose, starch and ethylcellulose. Lubricants that can be used include magnesium stearate or other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more esthetic in appearance or to help identify the product.

For intravenous (IV) use, a water soluble form of the antibiotic can be dissolved in one of the commonly used intravenous fluids and administered by infusion. Such fluids as, for example, physiological saline, Ringer's solution or 5% dextrose solution can be used.

For intramuscular preparations, a sterile formulation of a suitable soluble salt form of the antibiotic, for example the hydrochloride salt, can be dissolved and administered in a pharmaceutical diluent such as Water-for-Injection, physiological saline or 5% glucose solution. A suitable insoluble form of the antibiotic may be prepared and administered as a suspension in an aqueous base or a pharmaceutically acceptable oil base, e.g. an ester of a long chain fatty acid such as ethyl oleate.

For oral use, a sterile formulation of a suitable salt form of the antibiotic, for example, the hydrochloride salt, formulated in a diluent such as distilled or deionized water, is particularly useful.

Alternatively, the unit dosage form of the antibiotic can be a solution of the antibiotic or preferably a salt thereof in a suitable diluent in sterile, hermetically sealed ampoules. The concentration of the antibiotic in the unit dosage may vary, e.g. from about 1 percent to about 50 percent depending on the particular form of the antibiotic and its solubility and the dose desired by the physician.

In a further aspect, this invention provides a method for treating or controlling infectious diseases, especially those caused by gram-positive microorganisms, in animals. This method comprises administering to the animal an effective dose of an M43 complex. An effective dose is generally between about 0.5 and about 100 mg/kg of M43A or M43D or a pharmaceutically acceptable salt thereof; between about 1 and about 200 mg/kg of M43B or M43C or a pharmaceutically-acceptable salt thereof. A preferred dose is from about 10 to about 60 mg/kg of M43A or M43D compound; from about 20 to about 120 mg/kg of M43B or M43C. A typical daily dose for an adult human is from about 250 mg to about 1.0 g of M43A or M43D; from about 500 mg to about 2.0 g of M43B or M43C.

In practicing this method, the antibiotic can be administered in a single daily dose or in multiple doses per day. The treatment regime may require administration over extended periods of time, e.g., for several days or for from two to three weeks. The amount per administered dose or the total amount administered will depend on such factors as the nature and severity of the infection, the age and general health of the patient, the tolerance of the patient to the antibiotic and the microorganism or microorganisms involved in the infection.

A convenient method of practicing the treatment method is to administer the antibiotic via IV infusion. In this procedure a sterile formulation of a suitable soluble salt of the antibiotic is incorporated in a solution of a physiological fluid, such as 5% dextrose solution, and the resulting solution is infused slowly IV. Alternatively, the piggy-back method of IV infusion can be used.

In another embodiment, this invention relates to methods of increasing feed-utilization efficiency in poultry, swine, sheep and cattle, of promoting growth rates in cattle raised for meat production and of enhancing milk production in lactating ruminants. For increasing feed utilization efficiency and promoting growth, an M43 complex is administered orally in a suitable feed in an amount of from about 2 to about 200 grams per ton of total feed. For beef cattle, for example, a range of about 12 to 3000 mg/head/day is suitable. For enhancing milk production in lactating ruminants, oral administration of a daily amount of from about 0.04 to about 16 mg/kg of body weight (or about 25 to about 5000 mg/ruminant/day) is suggested.

The following examples are provided to illustrate this invention:

## Example 1

### Isolation of Antibiotic M43A, M43B and M43C from *N. orientalis* NRRL 2452

A. Separation from Vancomycin

Using the procedure in U.S. 3,067,099 (Example 2), fourteen 1200 gallon fermenters were processed to give a crude preparation in the hydrochloride salt form. This preparation was further separated on a Dowex 50—2X resin column, selectively eluting fractions containing M43A with a 2-percent aqueous ammonium formate solution (pH 9.6) as the eluent, to give a semi-purified preparation containing M43A, M43B and M43C.

B. Purification of M43A

A portion of the M43A-enriched material (1.0—1.3 g) was suspended in deionized water. The pH of the resulting suspension was adjusted to pH 3 with 10% aqueous phosphoric acid. The resulting solution, diluted to a volume of 10 ml, was passed through a .45-µm membrane filter (Millex—HA 0.45-µm filter unit, Millipore Corporation, Bedford, MA, 01730). The filtrate was separated in two-ml portions through a chromatography column (37 × 350 mm) containing LiChroprep® RP—18 (15—25 µm (Art. #13901, E. Merck, Darmstadt, Germany) as the stationary phase. The column was eluted with an aqueous acetonitrile gradient containing 12—20% of acetonitrile. The aqueous phase of the gradient contained triethylamine (0.2%) and was adjusted (prior to the acetonitrile addition) to pH 3 with 10% aqueous phosphoric acid. The column effluent was monitored by UV activity and fractions were combined accordingly. The M43A-containing fractions from eighteen such separations were combined. The acetonitrile was removed by evaporation under high vacuum, and the aqueous solution was concentrated to one-tenth of the original volume. The concentrated aqueous solution was loaded on a column (15 × 275 mm) containing freshly conditioned Diaion HP—20 resin (Mitsubishi Chemical Industries Limited, Tokyo 100, Japan). The column was extensively washed with water and then was eluted with water containing 25—50% methanol. The methanol was removed under high vacuum, and the resulting aqueous solution was evaporated to dryness. M43A was obtained as a dry amorphorus solid (0.6 g).

C. Separation of M43A, M43B and M43C

A portion (15 g) of material prepared as in Section A was dissolved in water (1.5 L), filtered, and applied to a CM-Sephadex C—25 ($NH_4^+$) column (glass, 6- × 77-cm, packed with 2 L of resin in water). The column was washed with water (2.5 L) at a rate of 5—10 ml/min to remove impurities. The column was then developed using a linear gradient of (a) 8 liters of water in the mixing chambers and (b) 8 liters of 2 M $NH_4HCO_3$ in the reservoir. Elution was monitored by assay against *Bacillus subtilis* in minimal media. Key fractions were also analyzed by analytical HPLC. The M43B- and M43C-containing fractions were combined, desalted on HP—20, concentrated and lyophilized to give 50 mg of enriched material. This material was applied to a reversed phase silica gel column (LP1—C—18) which was eluted with a $CH_3CN$ (13%):aqueous 0.05 M $KH_2PO_4$, pH 3.2 (87%) solvent system, eluting at a flow rate of 2.5-ml/min. Fractions were monitored by analytical HPLC. The M43B-containing fractions were combined, desalted and lyophilized to give 14.6 mg of antibiotic M43B. The M43C-containing fractions were combined, desalted and lyophilized to give 6.2 mg of M43C.

## Example 2

### Shake-flask Fermentation of *N. orientalis* NRRL 2450 to Produce M43A, M43B, M43C and M43D

A lyophilized pellet of *Nocardia orientalis* M43—05865 (NRRL 2450) is dispersed in 1—2 ml of sterilized water. This solution (<0.1 ml) is used to inoculate an agar slant having the following composition:

| Ingredient | Amount (g/L.) |
|---|---|
| Dextrin | 10 |
| Enzymatic hydrolysate of casein[a] | 2 |
| Beef extract | 1 |
| Yeast extract | 1 |
| Agar | 20 |
| Distilled water | q.s. to 1 liter |

[a] N—Z Amine A, Humko Sheffield Chemical, Lyndhurst NJ

The inoculated slant is incubated at 30°C for 4—6 days. The mature slant culture is covered with sterile distilled water and scraped with a loop to loosen the spores. The resulting spore suspension (1 ml) is used to inoculate 100 ml of a vegetative medium having the following composition:

| Ingredient | Amount (g/L.) |
|---|---|
| Glucose | 15 |
| Soybean meal | 15 |
| Cornsteep solids | 5 |
| $CaCO_3$ | 2 |
| NaCl | 5 |
| Tap $H_2O$ | q.s. to 1 liter |

The inoculated vegetative medium is incubated in a 500-ml Erlenmeyer flask for 24—48 hours at 30°C on a reciprocal shaker with a 2-inch stroke at 108 RPM or on a rotary shaker operating at 250 RPM.

This incubated vegetative medium (5 ml) is used to inoculate 100 ml of a sterilized (120°C for 30 minutes) production medium having the following composition:

| Ingredient | Amount (g/L.) |
|---|---|
| Glucose | 10 |
| Edible molasses | 20 |
| Peptone[a] | 5 |
| $CaCO_3$ | 2 |
| Tap $H_2O$ | q.s. to 1 liter |

[a] Bacto (Difco Laboratories, Detroit, MI)

The inoculated fermentation medium is incubated in a 500-ml Erlenmeyer flask at 25°—30°C for 72—96 hours on either a rotary shaker operating at 250 RPM or a reciprocal shaker operating at 108 strokes per minute. The pH of the uninoculated medium varies with the medium used for production, but the production media of Examples 2—5 have an initial pH range of 6.0 to 7.5 and a harvest pH range of 6.5 to 8.0.

B. Isolation of M43 Complex

Whole broth (2 L.), prepared as described in Section A, was filtered. The filtrate was treated with a cation exchange resin (Dowex 50W—X4, $H^+$, $NH_4^+$, pH 5.0), using 100-ml of resin and stirring batchwise for 30 minutes. The effluent was decanted and discarded. The resin was washed thoroughly with water, and the water wash was discarded. The resin was then eluted batchwise with 1N $NH_4OH$ (250 ml and 175 ml per

batch). The eluates were combined and concentrated under vacuum to a volume of about 50 ml. An aliquot (2 ml) was removed for assay, and the remaining concentrate was lyophilized to give 300 mg of M43 complex.

C. Separation of M43A by Analytical HPLC

A portion of M43 complex, prepared and described in Section B, was dissolved in water at a concentration of 10 mg/ml. The solution was adjusted to pH 2.3 to increase the solubility of the complex, and the suspension was then centrifuged. The supernatant was examined by analytical HPLC, using the following conditions:

Column: 4.6- × 250-mm stainless steel, prepacked by Altex
Packing: Ultrasphere ODS — 5 micron
Solvent: A — $CH_3CN$:0.05 M $KH_2PO_4$ (1:9) pH 3.2
B — $CH_3CN$:0.05 M $KH_2PO_4$ (2:8) pH 3.2
Gradient: 0 percent B for 15 min;,, 0 → 40 percent B for 15—35 min;, 40 percent B for 35—38 min;, re-equilibrate in 0 percent B until 45 min.
Flow Rate: 1.0 ml/min. Chart Speed: 120 secs/cm
Detection: LDC Spectromonitor III at 280 nm
Sensitivity: 0.1 AUFS
Pumps: LDC Constametric III
System controlled by LDC Chromatograph Control Module (CCM)
Sample volume: 10 µl

Peak areas were measured by the CCM; then the normalization method of integration was used to give area-percent values for each peak. M43 complex contained M43A (area percent = 39.1) and vancomycin (area percent 15.1) and many additional minor factors. Thus, the ratio of antibiotic M43A to vancomycin produced by *N. orientalis* NRRL 2450, as measured by this experiment, was 2.6:1.

D. Alternate Separation of M43A by Analytical HPLC

M43 complex is examined by analytical HPLC, using the following system:
Column: Beckman Ultrasphere (5 µ particle size), ODS, 25 cm
Mobile Phase: Solvent A: $CH_3CN$/TEAP (5:95)
Solvent B: $CH_3CN$/TEAP (2:3)
[TEAP = 0.5% aqueous triethylamine adjusted to pH 3 with conc. phosphoric acid]
Gradient: 9% B to 70% B over a 40-min period; then hold for 5 min. at 70% B
Flow Rate: 1.0 ml/min.
Detection: UV at 254 nm

| M43A Factor | Retention Time (min.) |
|---|---|
| A51568 factor B[a] | 5.92 |
| A51568 factor A | 8.96 |
| vancomycin | 12.23 |
| desvancosamine-A51568A | 17.59 |
| M43D | 19.96 |
| desvancosamine-vancomycin[a] | 20.38 |
| M43A | 24.26 |
| M43B[a] | 25.46 |
| M43C[a] | 29.58 |
| agluco-A51568A | 36.97 |
| aglucovancomycin | 37.72 |
| agluco-M43A | 39.79 |

[a] trace amount

E. Recommended Isolation Procedure for M43A

The following procedure is recommended for isolating antibiotic M43A from M43A complex:

I. Chromatograph M43A complex over highly porous polymer (Diaion HP—20, Mitsubishi Chemical Industries, Ltd., Tokyo, Japan):

1) Wash with water and discard the water wash;

2) Wash with 50% aqueous $CH_3CN$ and discard this wash;

3) Wash with 50% aqueous $CH_3CN$ containing 0.1% $CH_3COOH$; this eluate contains the M43A.

II. Chromatograph the M43A-containing eluate (3) over reversed-phase silica gel $C_{18}$ resin to separate M43A from the remaining factors.

III. Chromatograph the separated M43A over HP—20 polymer to desalt the antibiotic, giving pure antibiotic M43A.

F. Separation of M43B, M43C and M43D by Analytical HPLC

A portion of the M43 complex prepared as described in Section B is examined by analytical HPLC, using the following system:

Column: Beckman Ultrasphere (5 μ particle size), ODS, 25 cm

Mobile Phase: Solvent A: $CH_3CN$/TEAP (5:95)
Solvent B: $CH_3CN$/TEAP (2:3)

[TEAP = 0.5% aqueous triethylamine adjusted to pH 3 with conc. phosphoric acid]

Gradient: 9% B to 70% B over a 40-min period, then hold for 5 min. at 70% B

Flow Rate: 1.0 ml/min.

Detection: UV at 254 nm

| M43 Factors | Retention Time (min.) |
|---|---|
| A51568 factor B[a] | 5.92 |
| A51568 factor A | 8.96 |
| vancomycin | 12.23 |
| desvancosamine-A51568A | 17.59 |
| M43D | 19.96 |
| desvancosamine-vancomycin[a] | 20.38 |
| M43A | 24.26 |
| M43B[a] | 25.46 |
| M43C[a] | 29.58 |
| agluco A51568A | 36.97 |
| agluco-vancomycin | 37.72 |
| agluco-M43A | 39.79 |

[a] Trace amounts

G. Preparative Separation of M43D from Vancomycin

Vancomycin hydrochloride (1.5 g) was dissolved in deionized water. The resulting solution was diluted with deionized water to a volume of 10 ml. This solution was passed through a 0.45-μm membrane filter[1)]. The filtrate was chromatographed in 2-ml portions through a column (37 × 350 mm) containing Li Chroprep RP—18 (15—25 μm)[2)] as the stationary phase. The column was eluted with an aqueous acetonitrile gradient containing 7.5—20% (v/v) of acetonitrile. The aqueous phase of the gradient contained triethylamine (0.2%) and was adjusted (prior to the acetonitrile addition) to pH 3 with 10% aqueous phosphoric acid. The column effluent was monitored by UV activity and separated into fractions based on this activity. The M43D-containing fractions from forty such separations were combined. The acetonitrile

[1)] Millex-Ha 0.45 μm filter unit, Millipore Corporation, Bedford, MA 01730
[2)] Art. #13901, E. Merck Darmstadt-Germany

was removed by evaporation under high vacuum. Inorganic salts were removed by adsorption of M43D on Diaion HP—20 resin[3]. The HP—20 resin was washed with water and subsequently eluted with water containing methanol (25—50% methanol v/v). The methanol was removed under high vacuum, and the resulting aqueous solution was freeze-dried to give M43D as a amorphous white solid (0.107 g).

## Example 3

### Characteristics of M43A, M43B, M43C and M43D

M43A has an integer molecular weight of 1475 as determined by fast-atom-bombardment mass spectrometry. The molecular weight of M43A is 28 units higher than that of vancomycin (m.w. 1447).

The molecular structure of M43A, as shown in formula *3*, was deduced from X-ray diffraction studies on a closely-related derivative.

M43B has an integer molecular weight of 1476 as determined by fast-atom-bombardment mass spectrometry.

The molecular structure of M43B, as shown in formula *4*, is based on proton nuclear magnetic resonance (NMR) studies.

M43C has an integer molecular weight of 1332 as determined by fast-atom-bombardment mass spectrometry. The molecular weight of M43C is 143 units lower than that of M43A (m.w. 1475). This is consistent with the structural difference between M43A and M43C being the presence of an additional vancosamine in M43A.

The molecular structure of M43C, as shown in formula *5*, was deduced in part from $^{1}H$ NMR studies.

M43D has an integer molecular weight of 1461 as determined by fast-atom-bombardment mass spectrometry. The molecular weight of M43D is 14 units higher than that of vancomycin (m.w. 1447).

The molecular structure of M43D, as shown in formula *6*, was deduced in part from $^{1}H$ NMR studies.

The proton NMR assignments for M43A, M43C and M43D are summarized in Table IV. The chemical shifts listed in Table IV were obtained in DMSO-$d_6$ solution at 60°C and at 360 MHz proton frequency. The numbering scheme used is shown in formula *7*:

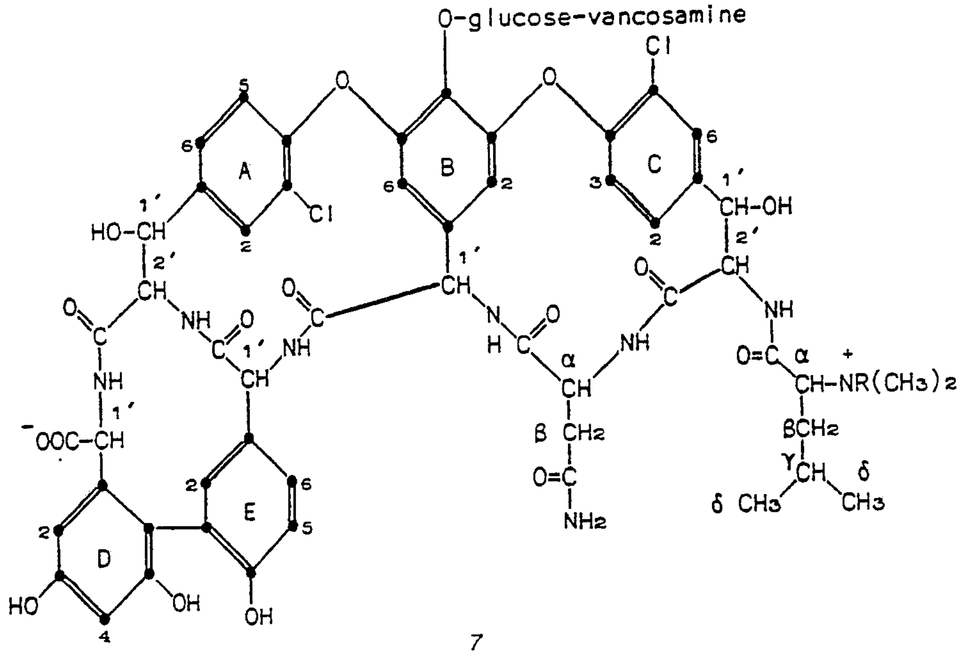

*7*

[3] Mitsubishi Chemical Industries Limited, Tokyo 100, Japan

Table IV: Proton NMR Assignments for M43A, M43C and M43D[a]

| Assignment | Chemical Shift | | |
|---|---|---|---|
| | M43A | M43C | M43D |
| A-NH | 6.46 | 6.45 | 6.51 |
| A-2' | 4.17 | 4.15 | 4.19 |
| A-1' | 5.14 | 5.21 | 5.12 |
| A-1'(OH) | 5.86 | | |
| A-2 | 7.88 | 7.86 | 7.85 |
| A-5 | 7.28 | 7.24 | 7.30 |
| A-6 | 7.45 | 7.46 | 7.45 |
| B-NH | 8.59 | 8.81 | 7.96 |
| B-1' | 5.77 | 5.76 | 5.68 |
| B-2 | 5.69 | 5.67 | 5.57 |
| B-6 | 5.19 | 5.21 | 5.24 |
| C-NH | 9.49 | 10.32 | 7.45 |
| C-2' | 4.84 | 4.92 | 4.85 |
| C-1' | 5.19 | 5.14 | 5.19 |
| C-1'(OH) | | | |
| C-2 | 7.55 | 7.52 | 7.52 |
| C-3 | 7.16 | 7.18 | 7.22 |
| C-6 | 7.61 | 7.43 | 7.30 |
| D-NH | 8.39 | 8.29 | 8.34 |
| D-1' | 4.44 | 4.41 | 4.45 |
| D-2 | 6.35 | 6.34 | 6.32 |
| D-4 | 6.38 | 6.45 | 6.38 |
| E-NH | 8.58 | 8.40 | |
| E-1' | 4.47 | 4.45 | |
| E-2 | 7.15 | 7.16 | |
| E-5 | 6.70 | 6.68 | |
| E-6 | 6.77 | 6.75 | |
| Asn-NH | ~6.3 | ~6.45 | 6.63 |
| Asn-α | 4.15 | 4.37 | 4.45 |
| Asn-β's | 2.73 and | 2.48 and | 2.16 and |
| " | 2.10 | 2.12 | 2.34 |

Table IV, cont'd.

| Assignment | Chemical Shift | | |
|---|---|---|---|
| | M43A | M43C | M43D |
| O<br>‖<br>Asn-C-$NH_2$ | 7.45 and | 7.29 | |
| " | 6.99 | 6.72 | |
| Leu-$\overset{+}{N}(CH_3)_3$ | 3.20 | 3.20 | 2.31 |
| Leu-α | 4.43 | 4.72 | 3.05 |
| Leu-β's | 1.92 and | 1.91 and | 1.57 and |
| | 1.65 | 1.59 | 1.39 |
| Leu-γ | 1.53 | 1.46 | 1.65 |
| Leu-δ's | 0.97 and | 0.93 | 0.87 and |
| | 0.90 | 0.87 | 0.87 |
| Glucose #1 | 5.33 | 5.32 | 5.35 |
| #2 | 3.59 | 3.40 | 3.60 |
| #3 | 3.46 | | 3.48 |
| #4 | | | 3.30 |
| #5 | | 3.24 | NA[b] |
| #6 | 3.57 and | 3.53 and | 3.69 and |
| | 3.70 | 3.70 | 3.58 |
| Vancosamine #1 | 5.27 | | 5.24 |
| #2 | 1.84 and | | 1.80 and |
| | 1.65 | | 1.62 |
| #3($CH_3$) | 1.28 | | 1.24 |
| #4 | | | NA |
| #5 | 4.64 | | 4.65 |
| #6($CH_3$) | 1.06 | | 1.07 |

[a]Phenols not yet assigned

[b]NA = not assigned

Example 4

Antibiotics M43A, M43B, M43C and M43D were prepared according to the method of Example 2, but using the following production medium:

| Ingredient | Amount (g/L.) |
|---|---|
| Glucose | 10 |
| Yeast | 5 |
| Distillers solubles | 5 |
| KCl | 4 |
| $CaCO_3$ | 1 |
| Tap $H_2O$ | q.s. to 1 liter |

Example 5

M43A, M43B, M43C and M43D were prepared by the method of Example 2, but using the following production medium:

| Ingredient | Amount |
|---|---|
| Casamino acids | 5 g/L |
| Dextrin | 5 g/L |
| Glycerol | 5 g/L |
| Blackstrap molasses | 10 g/L |
| Yeast | 5 g/L |
| $K_2HPO_4$ | 1 g/L |
| Mineral Stock | 5 ml |
| Tap $H_2O$ | q.s. to 1 liter |

Example 6

M43A, M43B, M43C and M43D were prepared by the method of Example 2, but using the following production medium:

| Ingredient | Amount (g/L.) |
|---|---|
| Soybean meal | 15 |
| Casein | 1 |
| $NaNO_3$ | 3 |
| Glucose syrup | 20 |
| Tap $H_2O$ | q.s. to 1 liter |

Example 7
M43A, M43C and M43D Tablet Formulation

Preparation of tablets containing 250 mg of M43A:

| Ingredient | Weight |
|---|---|
| M43A, M43C or M43D diphosphate | 282.9 of M43A or M43C or 283.2 mg of M43D |
| Microcrystalline cellulose | 101.1 mg |
| Croscarmellose sodium | 12.0 mg |
| Providone | 12.0 mg |
| Magnesium stearate | 3.0 mg |
| Stearic acid | 4.0 mg |
| Purified water | 0.16 ml |

Add M43A, M43C or M43D diphosphate, a portion of the microcrystalline cellulose and a portion of the croscarmellose sodium to a suitable container and blend until homogenous. Prepare a solution of Povidone in water, and add the Povidone solution to the blended powders. Granulate the resulting mixture, size if necessary and dry. Add the remaining microcrystalline cellulose and croscarmellose sodium to the dried mixture and blend. Add magnesium stearate and stearic acid, and blend the mixture. Compress the resulting powder blend into tablets with a theoretical weight of 415 mg. Each tablet contains M43A, M43C or M43D diphosphate equivalent to 250 mg of M43A, M43C or M43D.

Example 8
M43A, M43C or M43D Capsule Formulation

| Ingredient | Weight |
|---|---|
| M43A, M43C or M43D dihydrochloride | 262.2 mg |
| Corn starch flowable powder | 137.65 mg |
| Silicone fluid 350 centistokes | 2.75 mg |
| Corn starch | 147.1 mg |

Blend M43A, M43C or M43D dihydrochloride, starch flowable powder, silicone fluid 350 centistokes and starch powder in a suitable mixer until homogeneous. Fill into appropriate size hard gelatin capsules to a net fill weight of 550 mg. Each capsule contains M43A, M43C or M43D dihydrochloride equivalent to 250 mg of M43A, M43C or M43D.

Example 9
M43A, M43C or M43D Suspension Formulation

Prepare a sterile insoluble form of M43A, M43C or M43D by crystallization or precipitation. Mill or screen to a particle size suitable for suspension. Suspend the antibiotic in the following vehicle.

| Ingredient | Amount |
|---|---|
| Lecithin | 1% |
| Sodium citrate | 2% |
| Propylparaben | 0.015% |
| Water for Injection | q.s. to desired volume |

Sterilize the suspension. The suspension may be manufactured in bulk and filled into vials or may be prepared extemporaneously by adding the vehicle to the M43A, M43C or M43D in the vial.

**Claims for the Contracting States: BE CH DE FR GB IT LI NL SE**

1. Antibiotic M5-18260 or M43-05865 complex, or M43A, M43B, M43C or M43D, or a pharmaceutically-acceptable salt thereof, of the general formula:

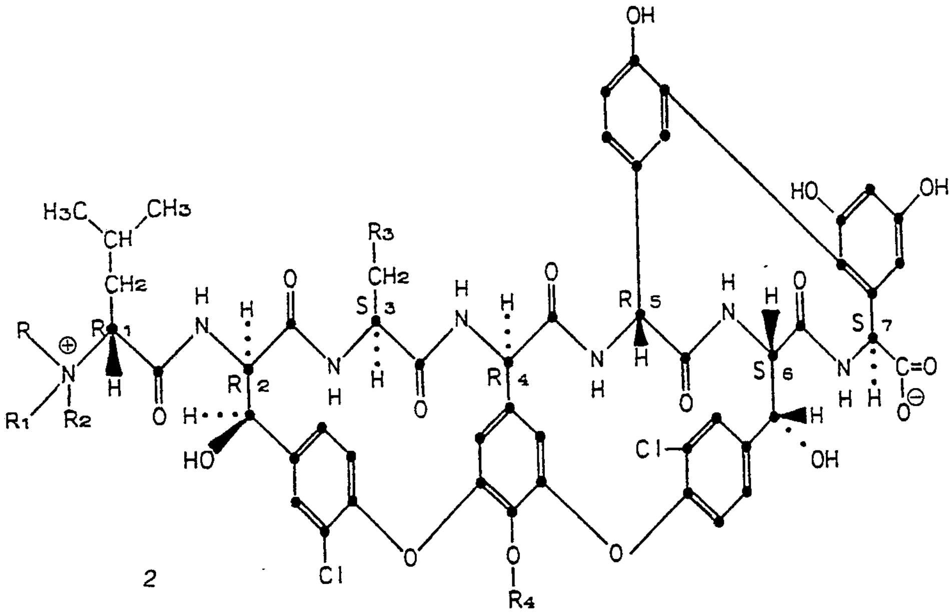

2

wherein:

R is H or methyl;
$R_1$ and $R_2$ are methyl;
$R_3$ is $CONH_2$ or COOH;
$R_4$ is glucosyl or vancosaminyl-O-glucosyl;
provided that when $R_4$ is glucosyl, then R must be methyl and $R_3$ must be $CONH_2$; or a pharmaceutically-acceptable salt thereof; and associated with less than 10% by weight of vancomycin.

2. M43A of the structure:

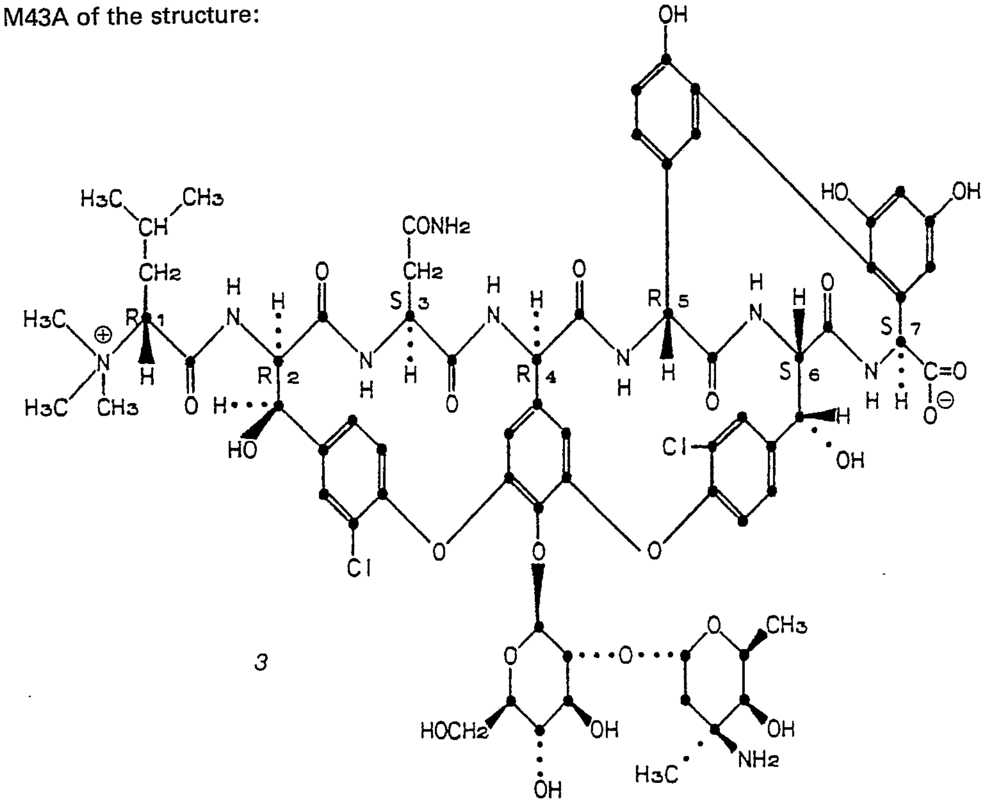

3

or a pharmaceutically-acceptable salt thereof, associated with less than 10% by weight of vancomycin.

3. M43B of the structure:

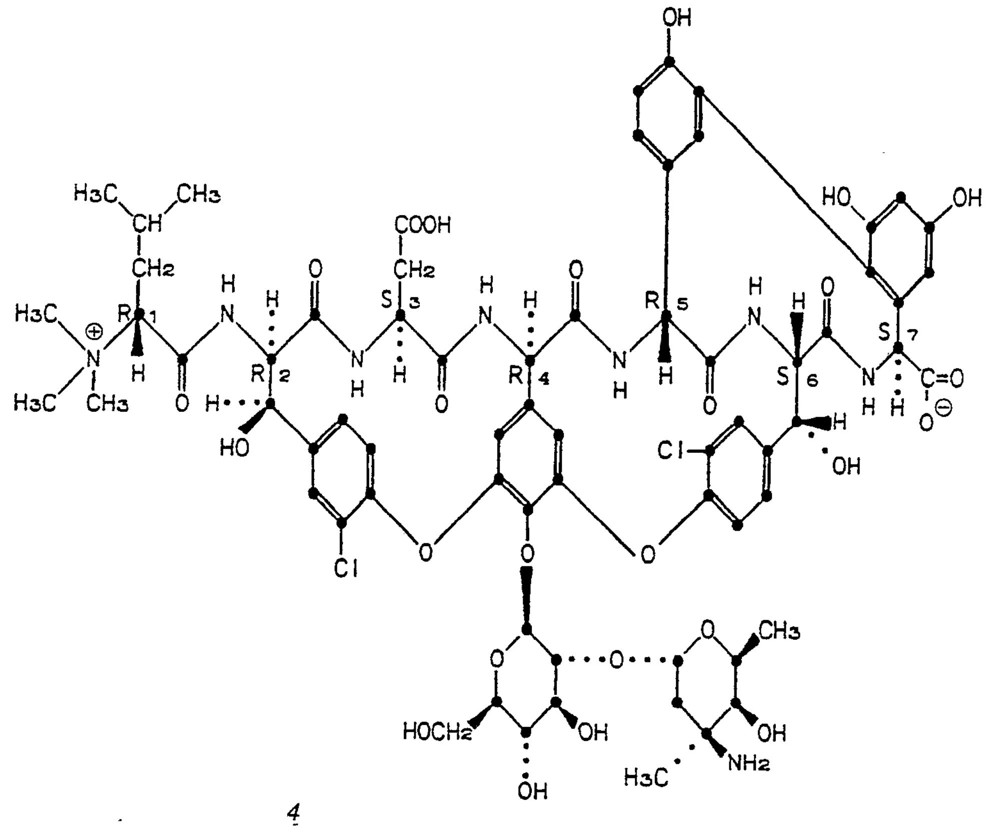

4

or a pharmaceutically-acceptable salt thereof, associated with less than 10% by weight of vancomycin.

4. M43C of the structure:

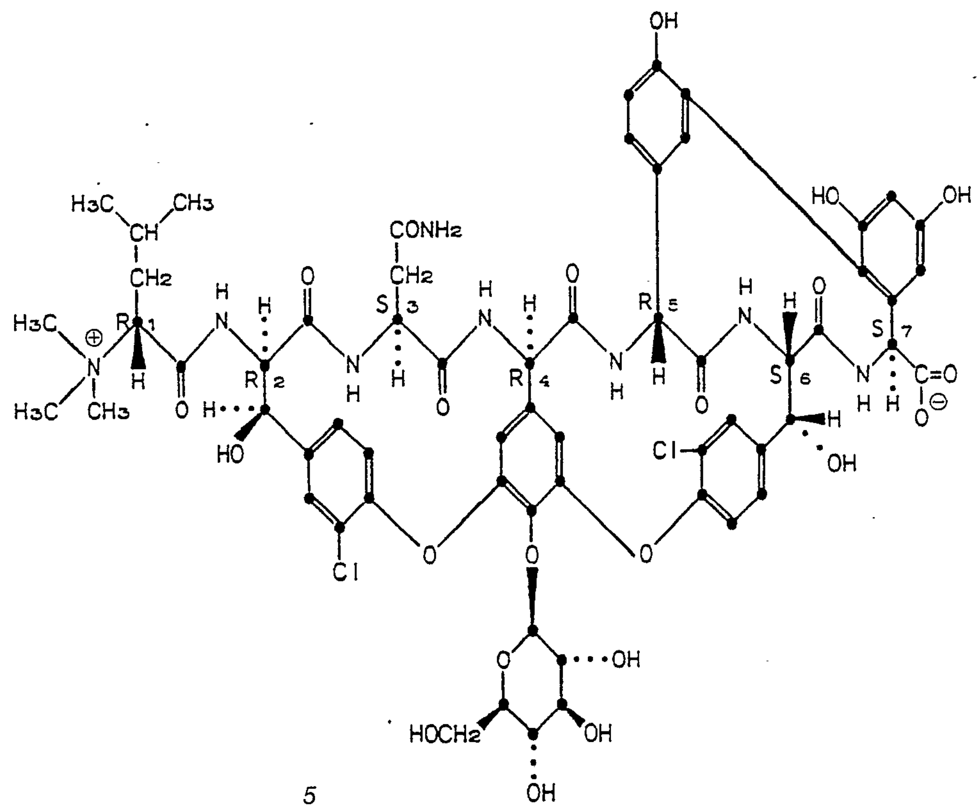

5

or a pharmaceutically-acceptable salt thereof, associated with less than 10% by weight of vancomycin.

5. M43D of the structure:

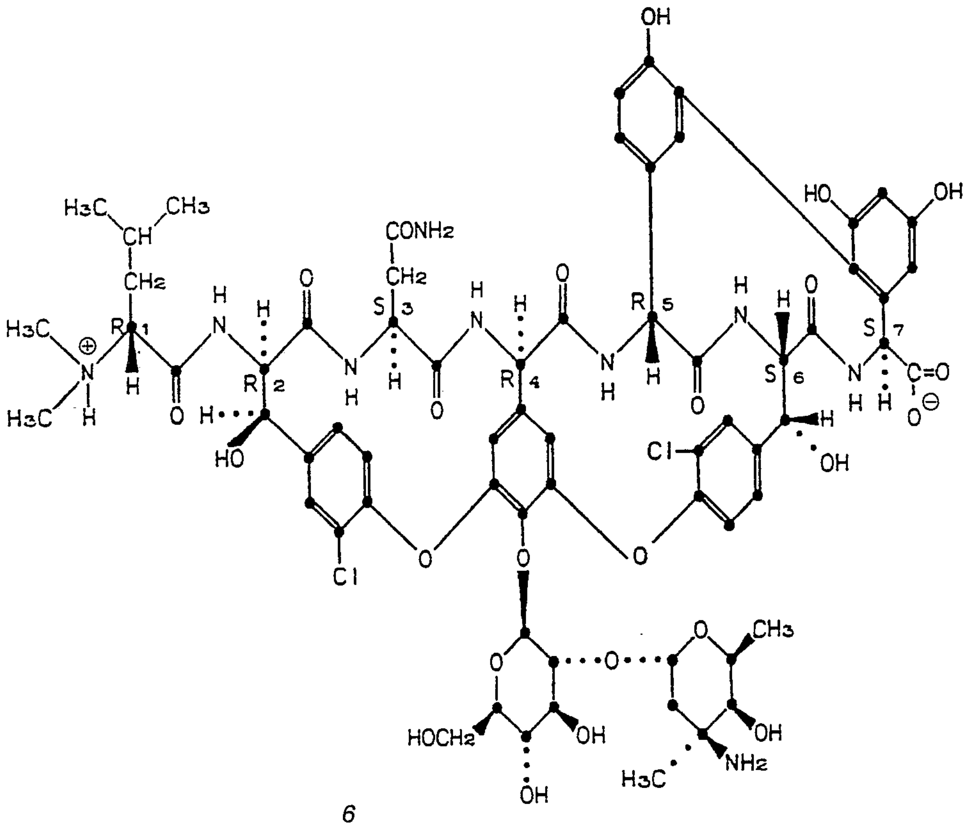

6

or a pharmaceutically-acceptable salt thereof, associated with less than 10% by weight of vancomycin.

6. A method of producing the M5-18260 or M43-05865 antibiotic complex or M43A, M43B, M43C or M43D as hereinbefore defined which comprises cultivating *Nocardia orientalis* NRRL 2450 or NRRL 2452, or an M5-18260 or M43-05865 producing variant, mutant or recombinant thereof, in a culture medium containing assimilable sources of carbon, nitrogen, and inorganic salts under submerged aerobic fermentation conditions.

7. A method according to claim 7 followed by separation of the complex from the culture medium and associated with less than 10% by weight of vancomycin.

8. A method according to claim 8 in which M43A, M43B, M43C or M43D is isolated from the complex and associated with less than 10% by weight of vancomycin.

9. Antibiotic M5-18260 or M43-05865 complex, or M43A, M43B, M43C or M43D, or a pharmaceutically-acceptable salt thereof, associated with less than 10% by weight of vancomycin, for use in pharmaceutical chemotherapy.

10. A pharmaceutical formulation which comprises as an active ingredient M43A, M43B, M43C or M43D, or a pharmaceutically-acceptable salt thereof, associated with less than 10% by weight of vancomycin and associated with one or more pharmaceutically-acceptable carriers or diluents therefor.

**Claims for the Contracting State: AT**

1. A process for preparing the M5-18260 or M43-05865 antibiotic complex or M43A, M43B, M43C or M43D of the general formula:

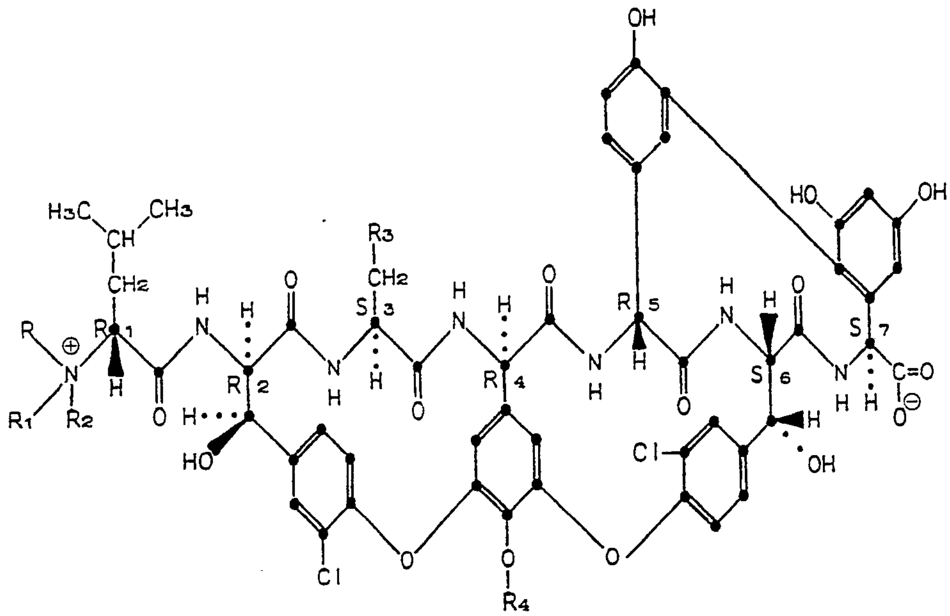

2

wherein:
R is H or methyl;
$R_1$ and $R_2$ are methyl;
$R_3$ is $CONH_2$ or COOH;
$R_4$ is glucosyl or vancosaminyl-O-glucosyl;
provided that when $R_4$ is glucosyl, then R must be methyl and $R_3$ must be $CONH_2$; or
a pharmaceutically-acceptable salt thereof; and
associated with less than 10% by weight of vancomycin which comprises cultivating *Nocardia orientalis* NRRL 2450 or NRRL 2452, or an M5-18260 or M43-05865 producing variant, mutant or recombinant thereof, in a culture medium containing assimilable sources of carbon, nitrogen, and inorganic salts under submerged aerobic fermentation conditions.

2. A process according to claim 1 followed by separation of the complex from the culture medium and associated with less than 10% by weight of vancomycin.

3. A process according to claim 2 in which M43A, M43B, M43C or M43D is isolated from the complex and associated with less than 10% by weight of vancomycin.

4. A process according to claim 3 for preparing M43A of the structure

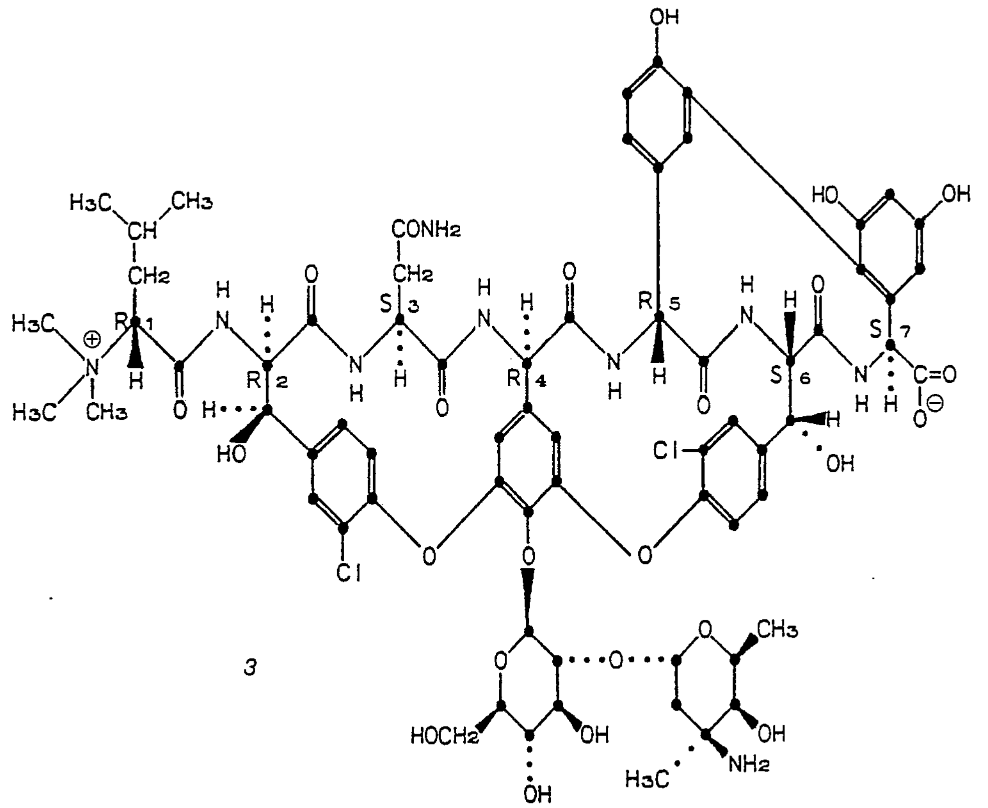

3

or a pharmaceutically-acceptable salt thereof, associated with less than 10% by weight of vancomycin.

5. A process according to claim 3 for preparing M43B of the structure:

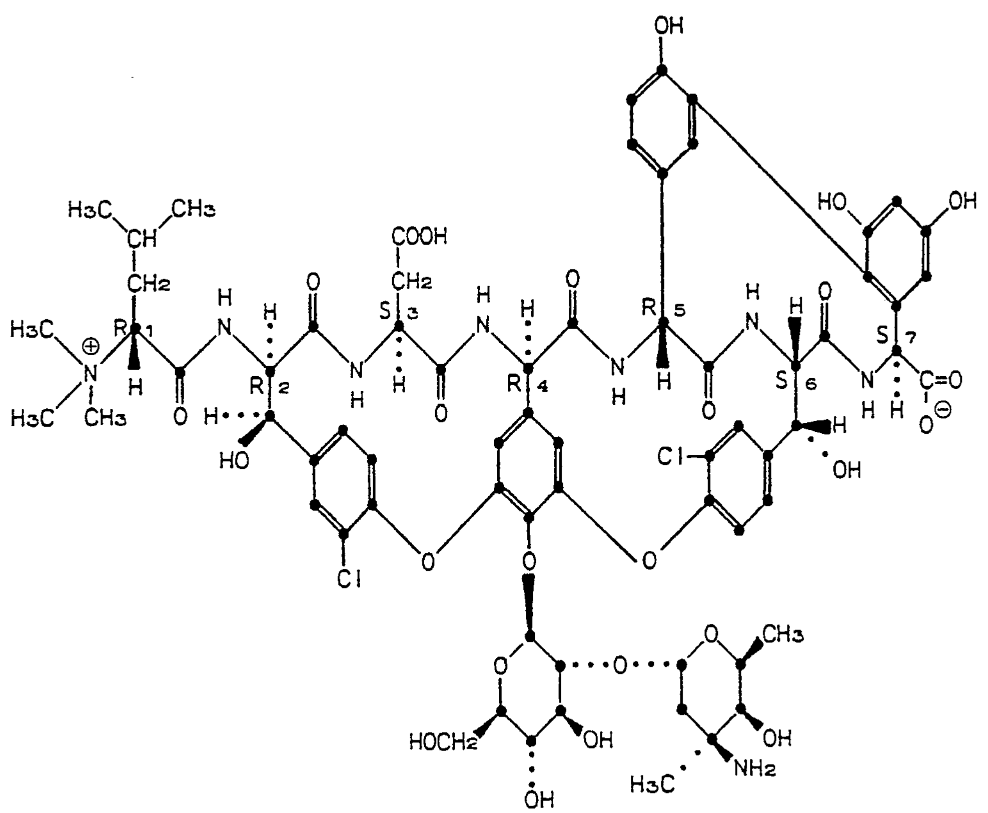

4

or a pharmaceutically-acceptable salt thereof, associated with less than 10% by weight of vancomycin.

6. A process according to claim 3 for preparing M43C of the structure:

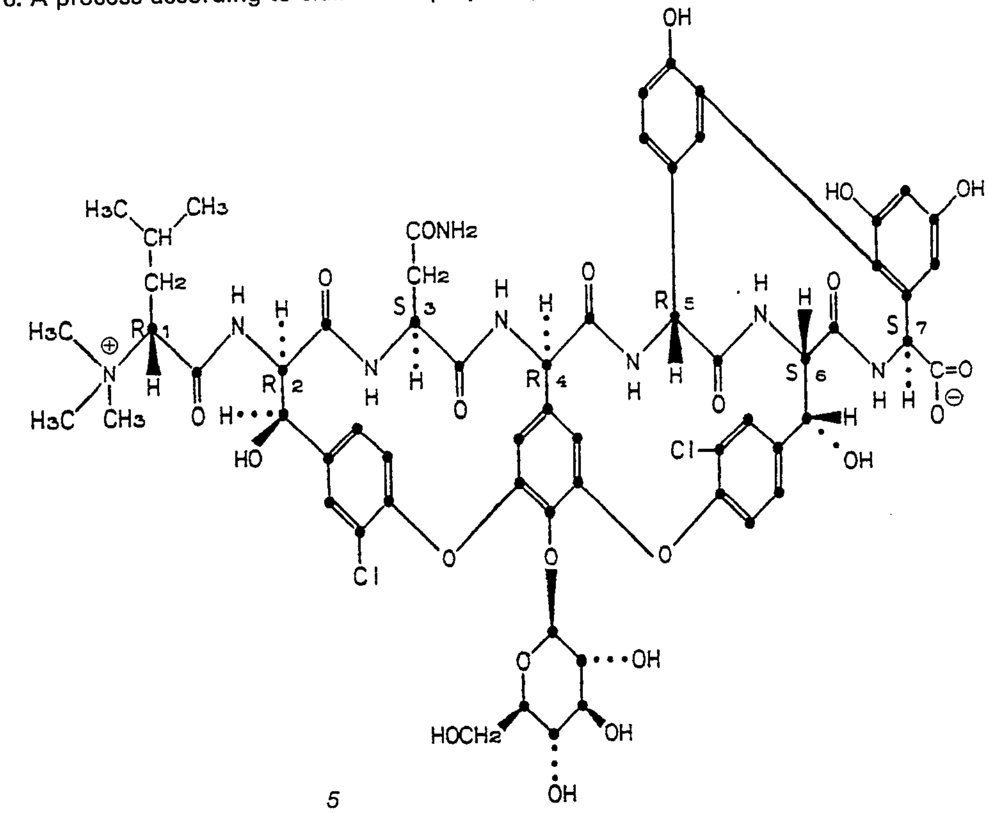

5

or a pharmaceutically-acceptable salt thereof, associated with less than 10% by weight of vancomycin.

7. A process according to claim 3 for preparing M43D of the structure:

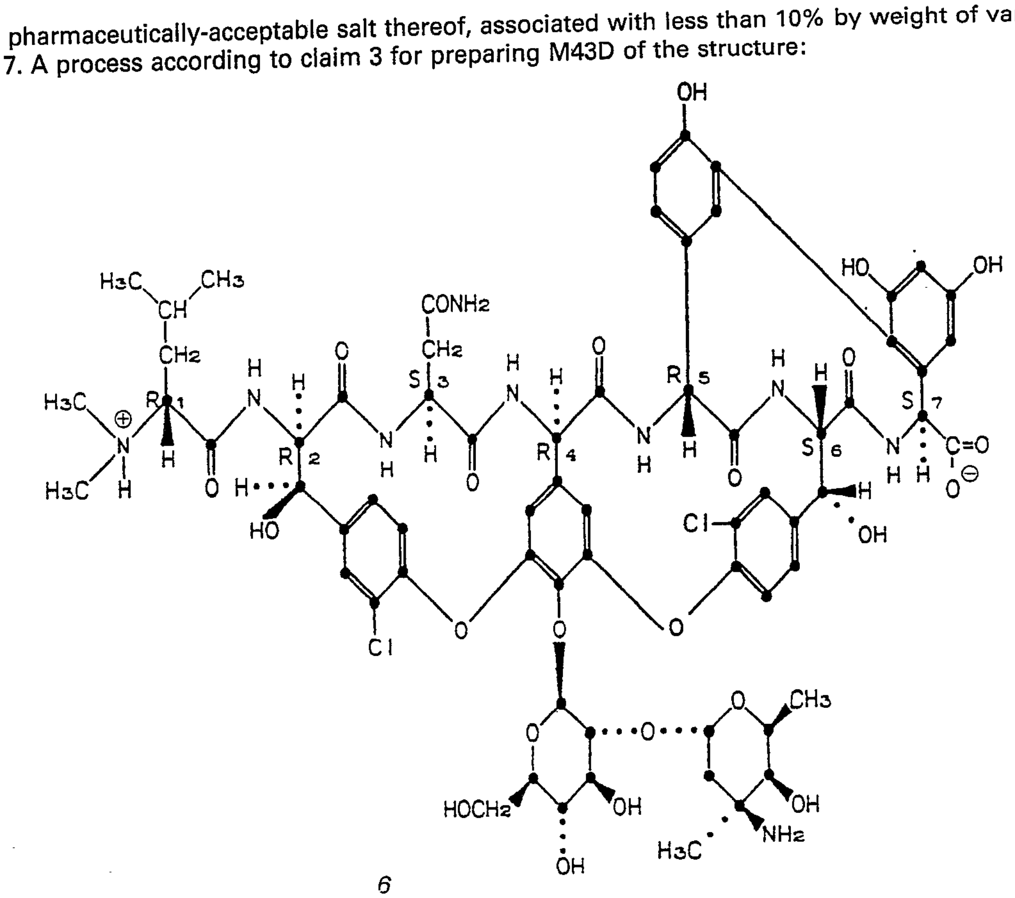

6

or a pharmaceutically-acceptable salt thereof, associated with less than 10% by weight of vancomycin.

**Patentansprüche für die Vertragsstaaten: BE CH DE FR GB IT LI NL SE**

1. Antibiotikumkomplex M5-18260 oder M43-05865 oder Antibiotikum M43A, M43B, M43C oder M43D oder ein pharmazeutisch annehmbares Salz hiervon der allgemeinen Formel

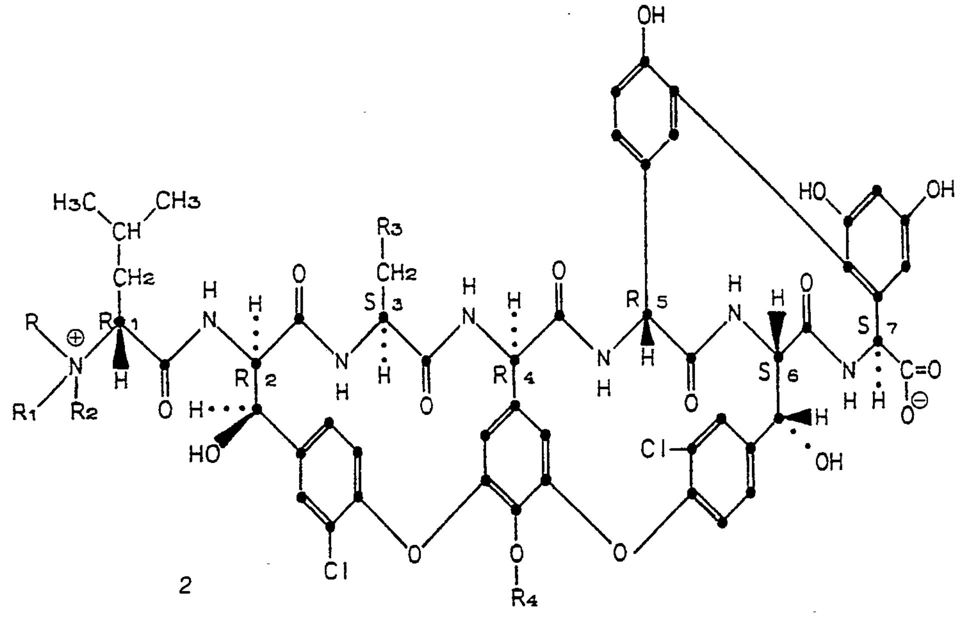

2

worin

R für H oder Methyl steht,

$R_1$ und $R_2$ Methyl sind,

$R_3$ für $CONH_2$ oder COOH steht,

$R_4$ Glucosyl oder Vancosaminyl-O-glucosyl ist,

mit der Maßgabe, daß R Methyl sein muß und $R_3$ für $CONH_2$ stehen muß, falls $R_4$ Glucosyl ist,

oder pharmazeutisch annehmbare Salze hiervon, wobei in diesem Komplex weniger als 10 Gewichtsprozent Vancomycin vorhanden sind.

2. Antibiotikum M43A der Struktur

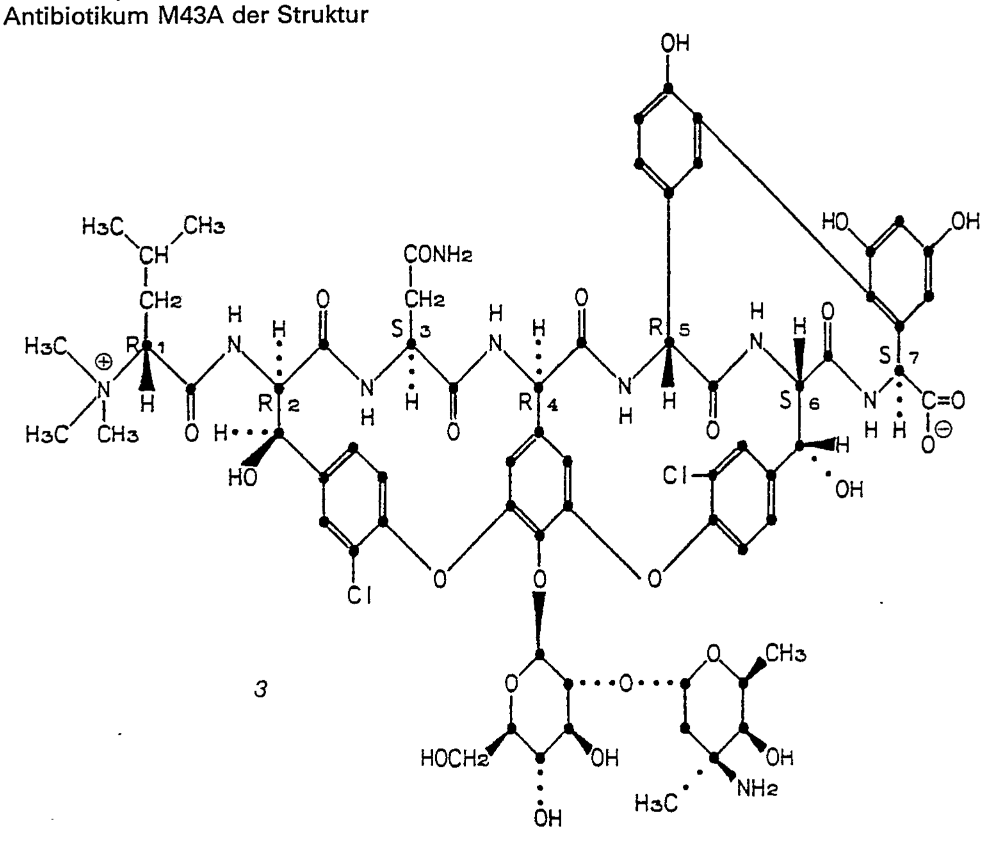

3

oder ein pharmazeutisch annehmbares Salz hiervon, das weniger als 10 Gewichtsprozent Vancomycin enthält.

3. Antibiotikum M43B mit der Struktur

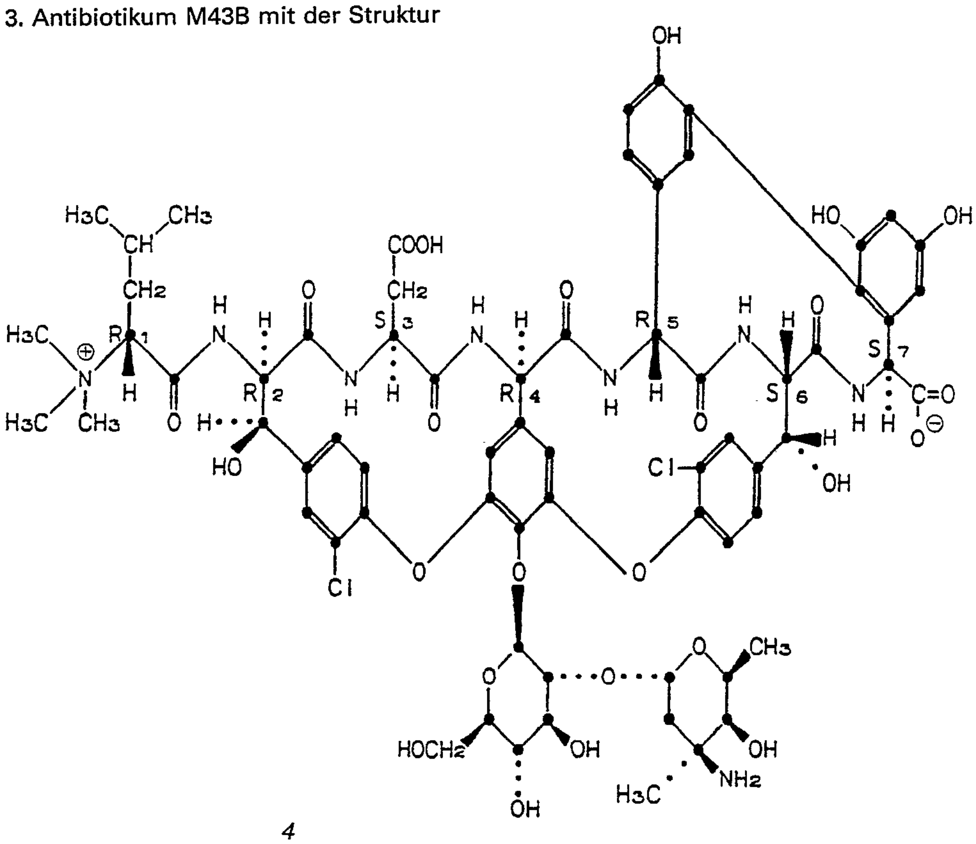

4

oder ein pharmazeutisch annehmbares Salz hiervon, das weniger als 10 Gewichtsprozent Vancomycin enthält.

4. Antibiotikum M43C mit der Struktur

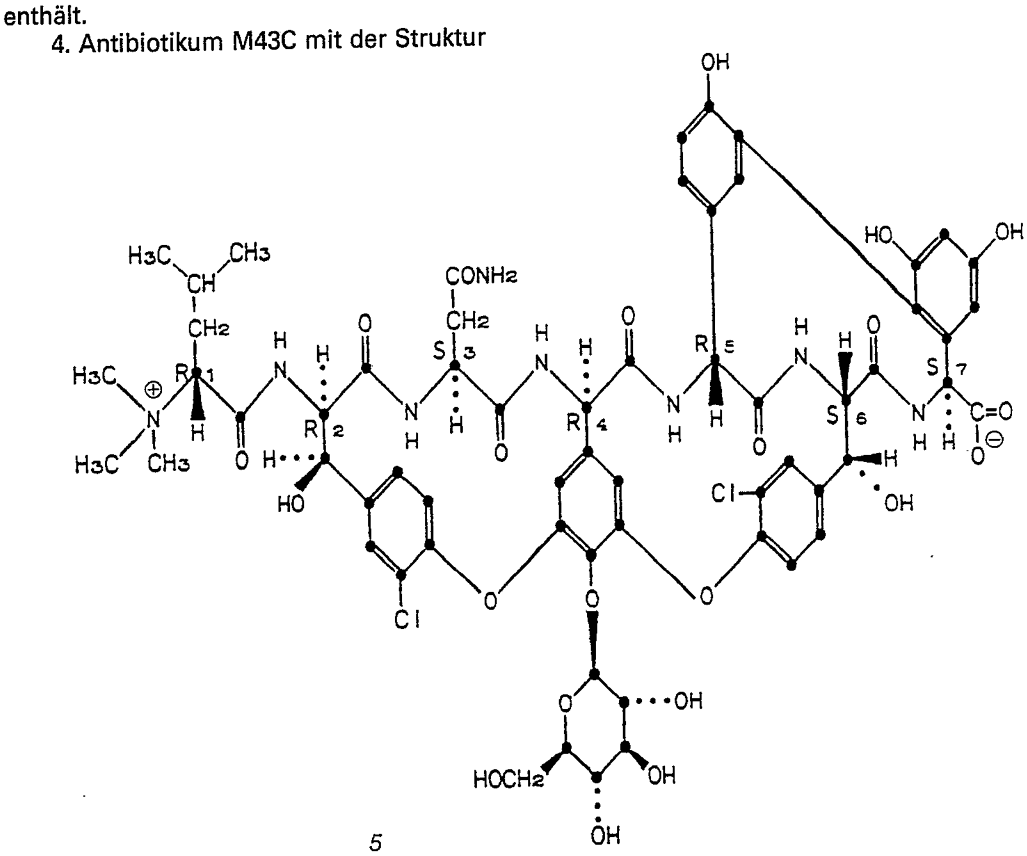

5

oder ein pharmazeutisch annehmbares Salz hiervon, das weniger als 10 Gewichtsprozent Vancomycin enthält.

5. Antibiotikum M43D mit der Struktur

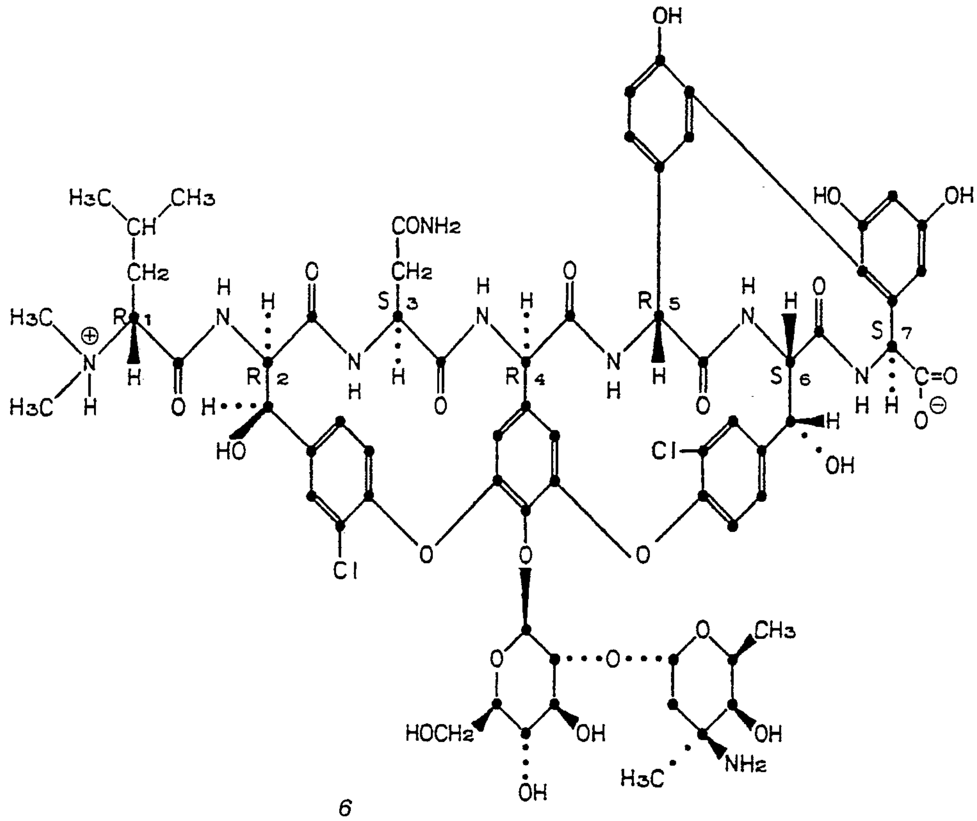

6

oder ein pharmazeutisch annehmbares Salz hiervon, das weniger als 10 Gewichtsprozent Vancomycin enthält.

6. Verfahren zur Herstellung des Antibiotikumkomplexes M5-18260 oder M43-05865 oder der Antibiotika M43A, M43B, M43C, oder M43D gemäß obiger Definition, dadurch gekennzeichnet, daß Nocardia orientalis NRRL 2450 oder NRRL 2452 oder eine M5-18260 oder M43-05865 produzierende Variante, Mutante oder Rekombinante hiervon in einem Kulturmedium, das assimilierbare Quellen für Kohlenstoff, Stickstoff und anorganische Salze enthält, unter submersen aeroben Fermentationsbedingungen gezüchtet wird.

7. Verfahren nach Anspruch 6, dadurch gekennzeichnet, daß der Komplex vom Kulturmedium abgetrennt wird und weniger als 10 Gewichtsprozent Vancomycin enthält.

8. Verfahren nach Anspruch 7, dadurch gekennzeichnet, daß die Antibiotika M43A, M43B, M43C oder M43D aus dem Komplex isoliert werden und weniger als 10 Gewichtsprozent Vancomycin enthalten.

9. Antibiotikumkomplex M5-18260 oder M43-05865 oder Antibiotika M43A, M43B, M43C oder M43D oder pharmazeutisch annehmbare Salze hiervon mit jeweils weniger als 10 Gewichtsprozent Vancomycin zur Verwendung in der pharmazeutischen Chemotherapie.

10. Pharmazeutische Formulierung, dadurch gekennzeichnet, daß sie ein Antibiotikum M43A, M43B, M43C oder M43D oder ein pharmazeutisch annehmbares Salz hiervon als Wirkstoff mit jeweils weniger als 10 Gewichtsprozent Vancomycin in Kombination mit einem oder mehreren pharmazeutisch annehmbaren Trägern oder Verdünnungsmitteln hierfür enthält.

**Patentansprüche für den Vertragsstaat: AT**

1. Verfahren zur Herstellung des Antibiotikumkomplexes M5-18260 oder M43-05865 oder der Antibiotika M43A, M43B, M43C oder M43D der allgemeinen Formel

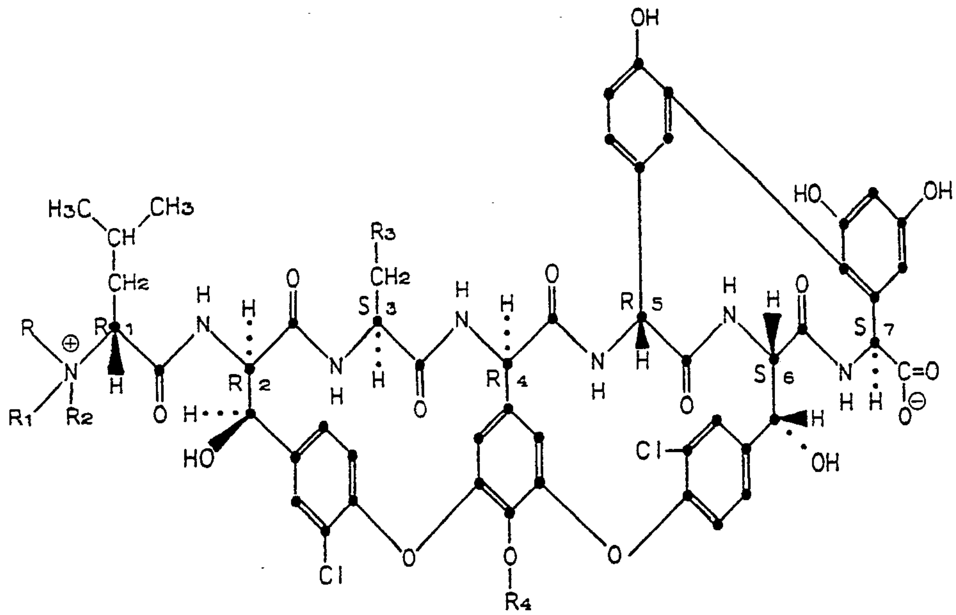

2

worin

R für H oder Methyl steht,

$R_1$ und $R_2$ Methyl sind,

$R_3$ für $CONH_2$ oder COOH steht,

$R_4$ Glucosyl oder Vancosaminyl-O-glucosyl ist,

mit der Maßgabe, daß R Methyl sein muß und $R_3$ für $CONH_2$ stehen muß, falls $R_4$ Glucosyl ist,

oder eines pharmazeutisch annehmbaren Salzes hiervon mit jeweils weniger als 10 Gewichtsprozent Vancomycin, dadurch gekennzeichnet, daß Nocardia orientalis NRRL 2450 oder NRRL 2452 oder eine M5-18260 oder M43-05865 produzierende Variante, Mutante oder Rekombinante hiervon in einem Kulturmedium, das assimilierbare Quellen für Kohlenstoff, Stickstoff und anorganische Salze enthält, unter submersen aeroben Fermentationsbedingungen gezüchtet wird.

2. Verfahren nach Anspruch 1, dadurch gekennzeichnet, daß der Komplex vom Kulturmedium abgetrennt wird und weniger als 10 Gewichtsprozent Vancomycin enthält.

3. Verfahren nach Anspruch 2, dadurch gekennzeichnet, daß die Antibiotika M43A, M43B, M43C oder M43D aus dem Komplex isoliert werden und jeweils weniger als 10 Gewichtsprozent Vancomycin enthalten.

4. Verfahren nach Anspruch 3, dadurch gekennzeichnet, daß das Antibiotikum M43A mit der Struktur

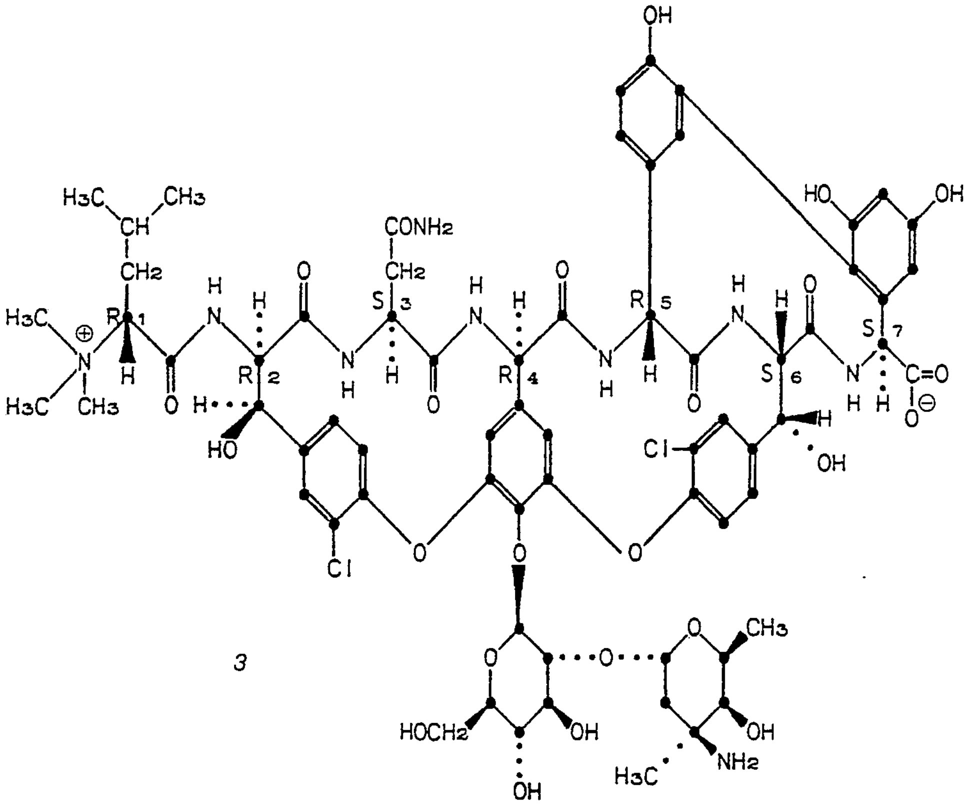

3

oder ein pharmazeutisch annehmbares Salz hiervon mit jeweils weniger als 10 Gewichtsprozent Vancomycin hergestellt wird.

5. Verfahren nach Anspruch 3, dadurch gekennzeichnet, daß das Antibiotikum M43B mit der Struktur

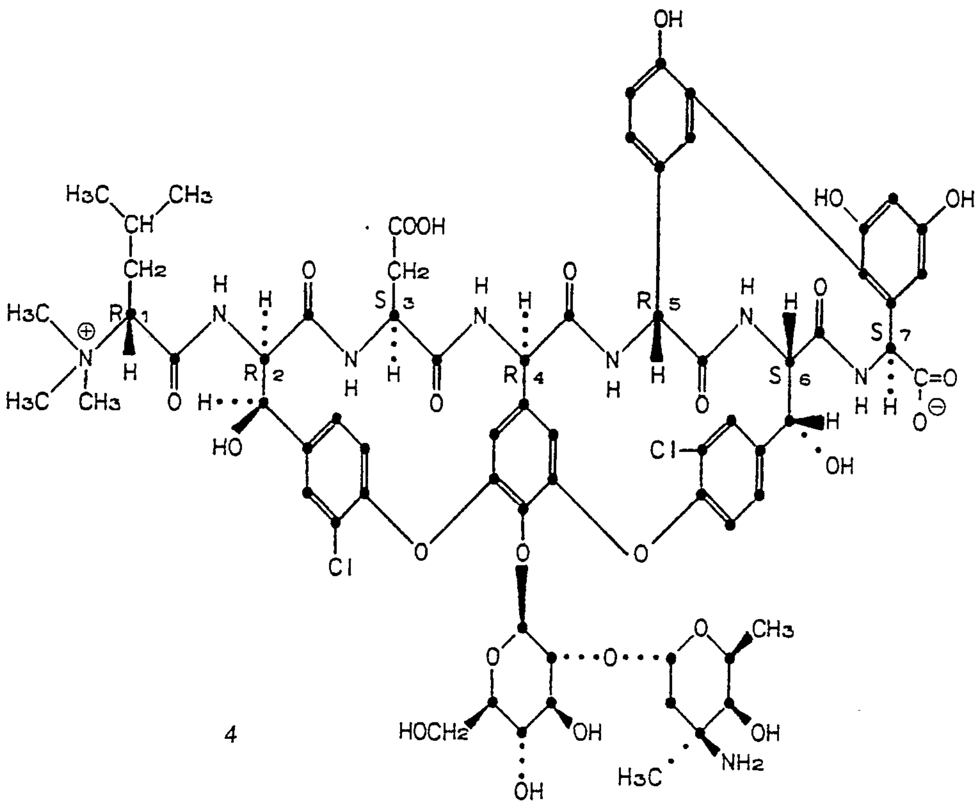

4

oder ein pharmazeutisch annehmbares Salz hiervon mit jeweils weniger als 10 Gewichtsprozent Vancomycin hergestellt wird.

6. Verfahren nach Anspruch 3, dadurch gekennzeichnet, daß das Antibiotikum M43C mit der Struktur

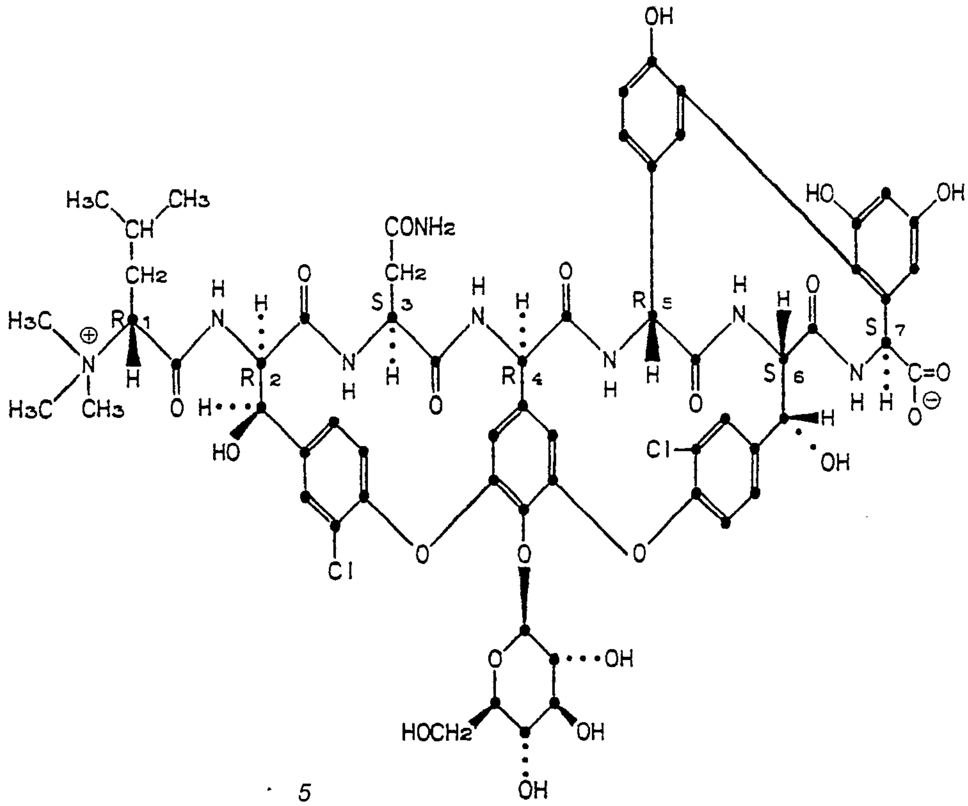

5

oder ein pharmazeutisch annehmbares Salz hiervon mit jeweils weniger als 10 Gewichtsprozent Vancomycin hergestellt wird.

7. Verfahren nach Anspruch 3, dadurch gekennzeichnet, daß das Antibiotikum M43D mit der Struktur

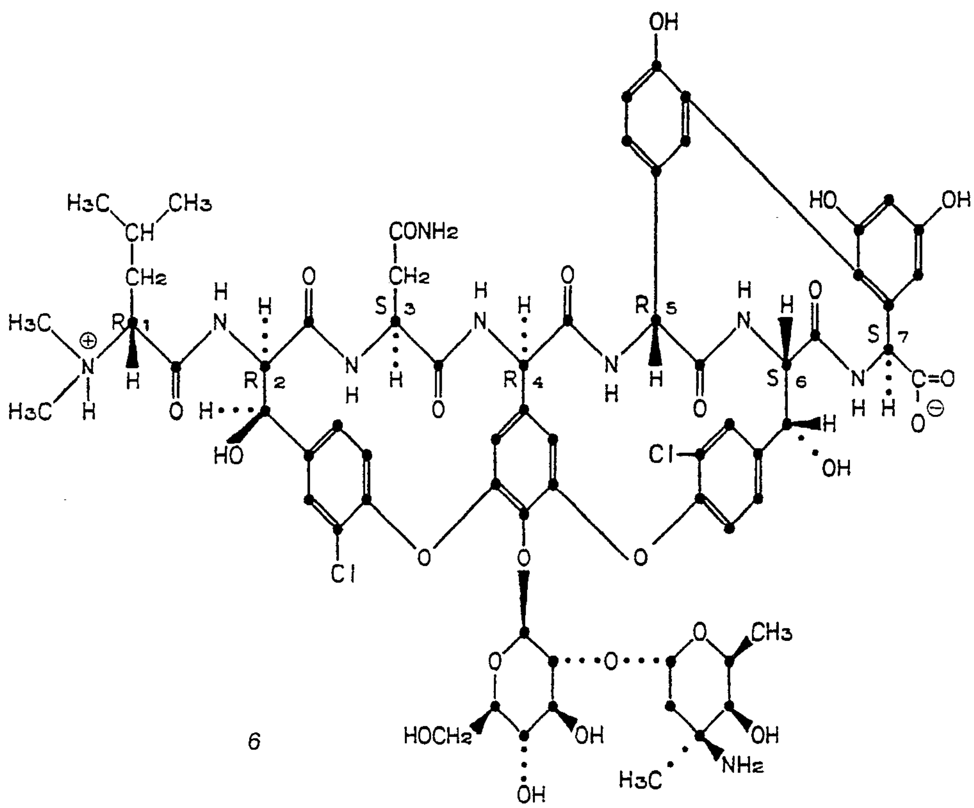

6

oder ein pharmazeutisch annehmbares Salz hiervon mit jeweils weniger als 10 Gewichtsprozent Vancomycin hergestellt wird.

**Revendications pour les Etats contractants: BE CH DE FR GB IT LI NL SE**

1. Complexe antibiotique M5-18260 ou M43-05865, ou antibiotique M43A, M43B, M43C ou M43D, ou encore un de leurs sels pharmaceutiquement acceptable, répondant à la formule générale:

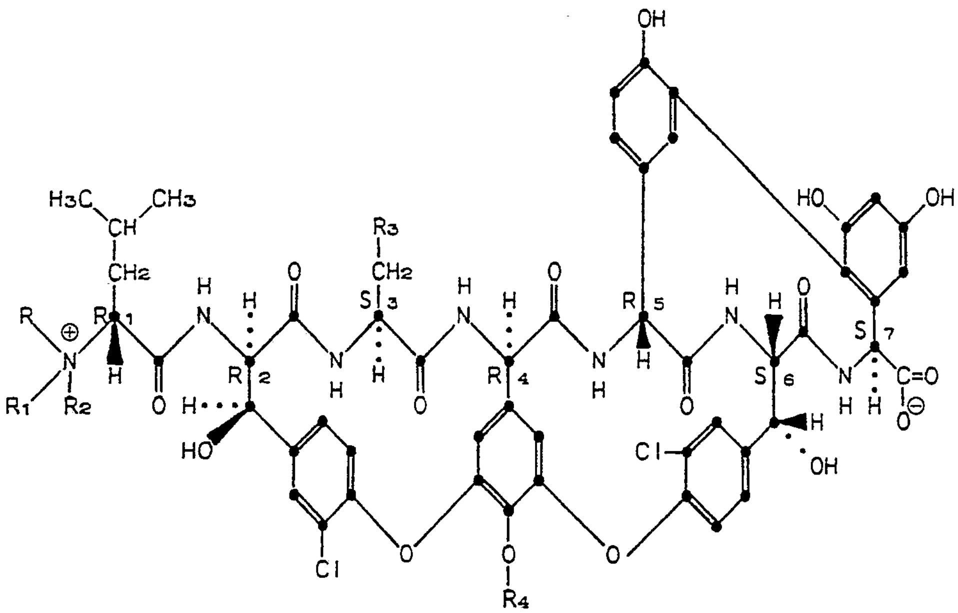

2

dans laquelle

R représente un atome d'hydrogène ou un groupe méthyle;

$R_1$ et $R_2$ représentent chacun un groupe méthyle;

$R_3$ représente $CONH_2$ ou COOH;

$R_4$ représente un groupe glucosyle ou un groupe vancosaminyl-O-glucosyle;

avec cette réserve que, lorsque $R_4$ représente un groupe glucosyle, alors R doit représenter un groupe méthyle et $R_3$ doit représenter $CONH_2$; ou

un de leurs sels pharmaceutiquement acceptable; et

en association avec moins de 10% en poids de vancomycine.

2. Antibiotique M43A de formule:

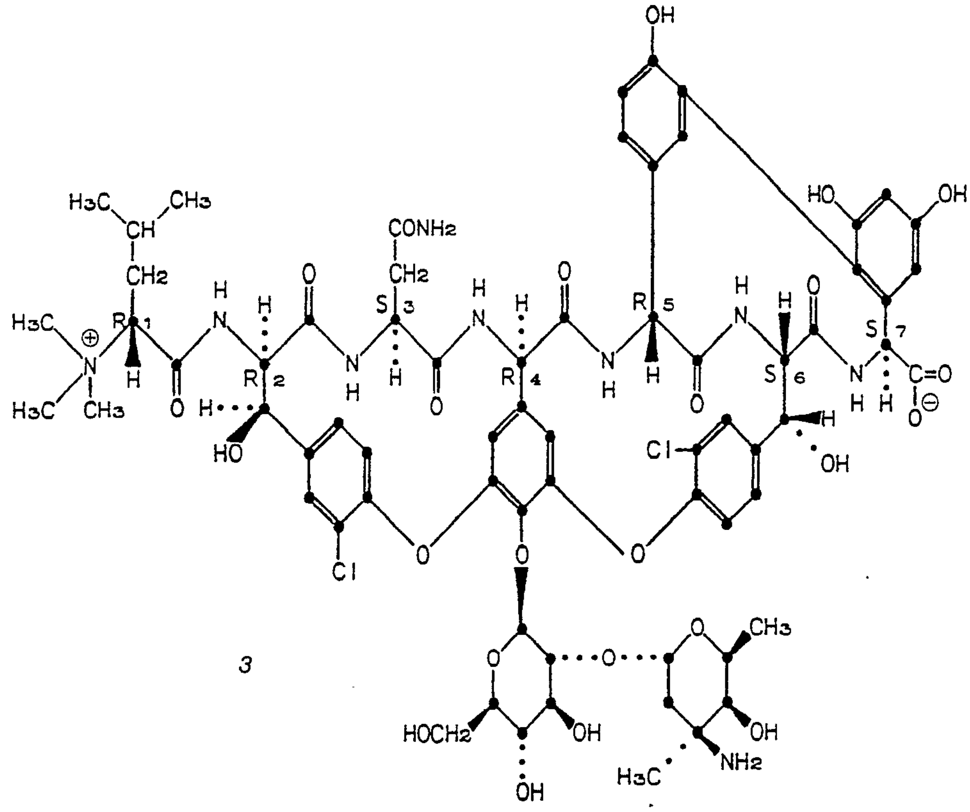

3

ou un de ses sels pharmaceutiquement acceptable, en association avec moins de 10% en poids de vancomycine.

3. Antibiotique de structure:

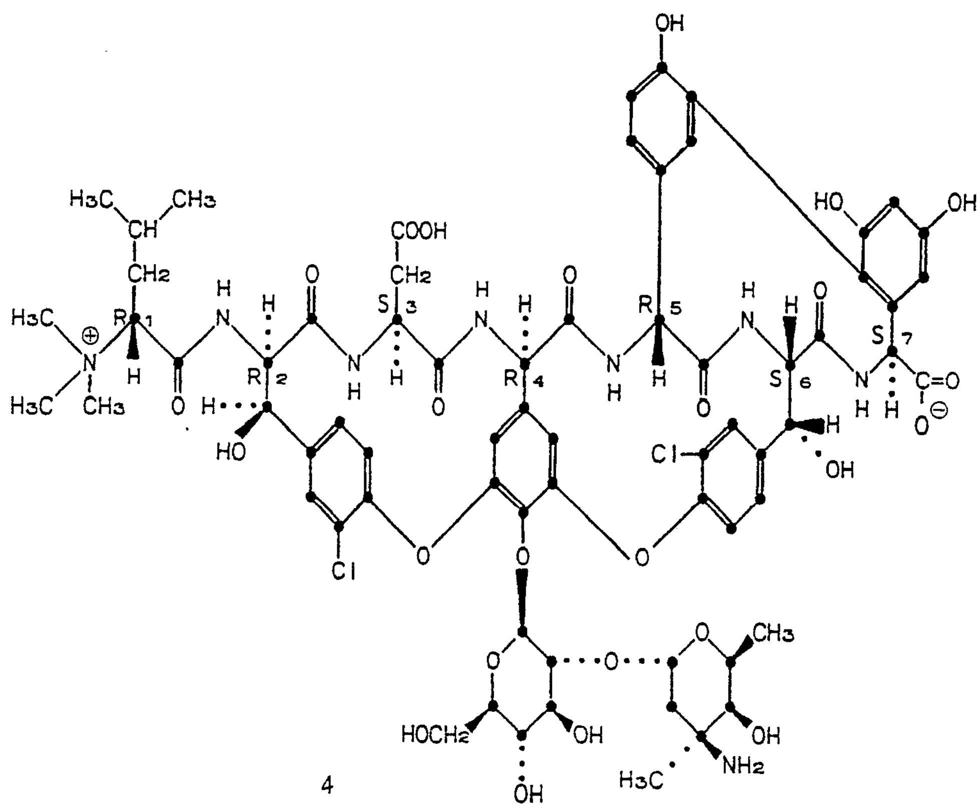

4

ou un de ses sels pharmaceutiquement acceptable, en association avec moins de 10% en poids de vancomycine.

4. Antibiotique M43C de structure:

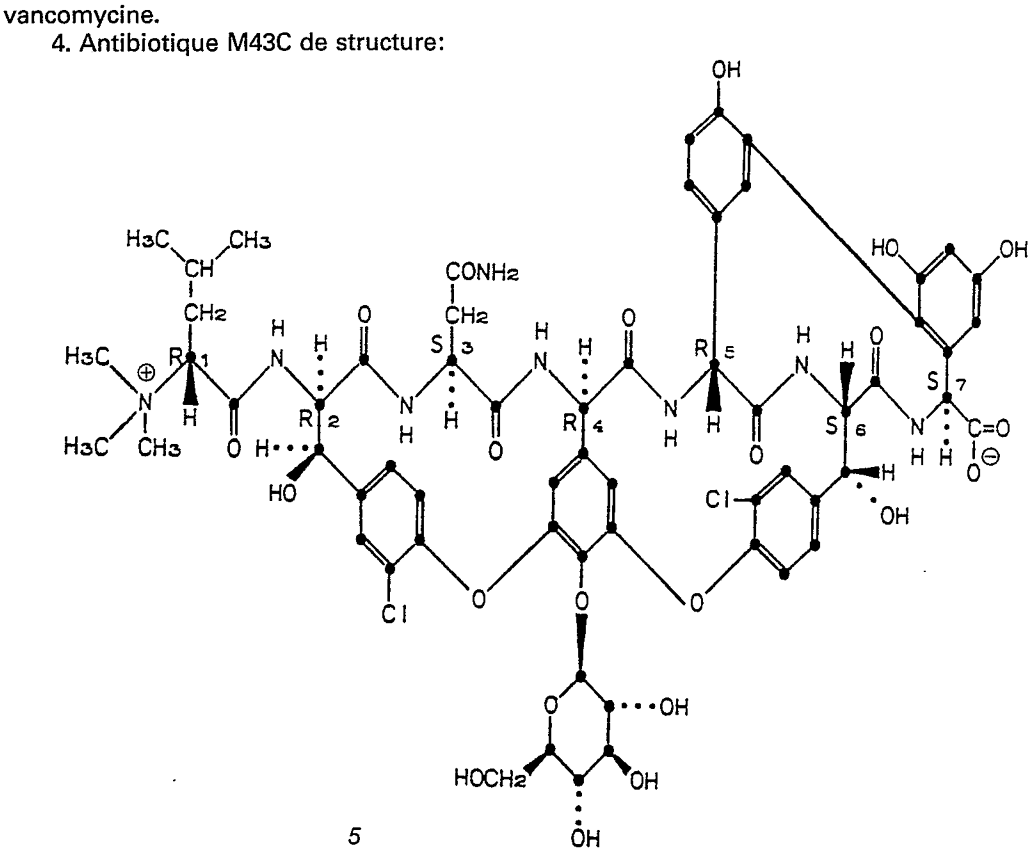

5

ou un de ses sels pharmaceutiquement acceptable, en association avec moins de 10% en poids de vancomycine.

5. Antibiotique M43D de structure:

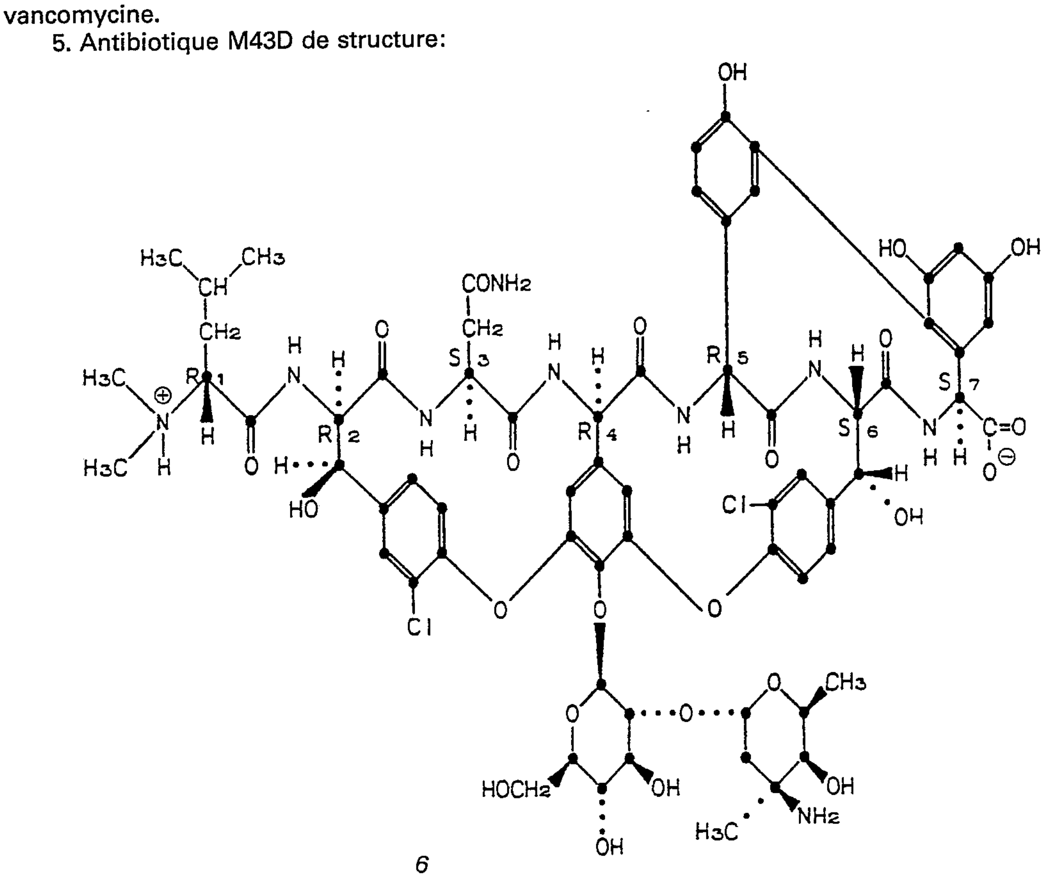

6

ou un de ses sels pharmaceutiquement acceptable, en association avec moins de 10% en poids de vancomycine.

6. Procédé de préparation du complexe antibiotique M5-18260 ou M43-05865, ou de l'antibiotique M43A, M43B, M43C ou M43D, tels que définis ci-dessus, ce procédé consistant à cultiver *Nocardia orientalis* NRRL 2450 ou NRRL 2452, ou un de ses variant, mutant ou recombinant producteur de M5-18260 ou de M43-05865, dans un milieu de culture contenant des sources assimilables de carbone, d'azote, et de sels inorganiques, dans des conditions de fermentation aérobie submergée.

7. Procédé selon la revendication 6, ce procédé étant suivi de la séparation du complexe hors du milieu de culture et de la mise en association avec moins de 10% en poids de vancomycine.

8. Procédé selon la revendication 7, procédé dans lequel on isole l'antibiotique M43A, M43B, M43C ou M43D, hors du complexe, tandis qu'on l'associe avec moins de 10% en poids de vancomycine.

9. Complexe antibiotique M5-18260 ou M43-05865, ou antibiotique M43A, M43B, M43C ou M43D, ou encore un de leurs sels pharmaceutiquement acceptable, en association avec moins de 10% en poids de vancomycine, pour l'utilisation en chimiothérapie pharmaceutique.

10. Formulation pharmaceutique comprenant, comme ingrédient actif, l'antibiotique M43A, M43B, M43C ou M43D, ou un de leurs sels pharmaceutiquement acceptable, en association avec moins de 10% en poids de vancomycine et en association avec un ou plusieurs supports ou diluants pharmaceutiquement acceptables, destinés à cet effet.

**Revendications pour l'Etat contractant: AT**

1. Procédé de préparation du complexe antibiotique M5-18260 ou M43-05865, ou de l'antibiotique M43A, M43B, M43C ou M43D de formule générale:

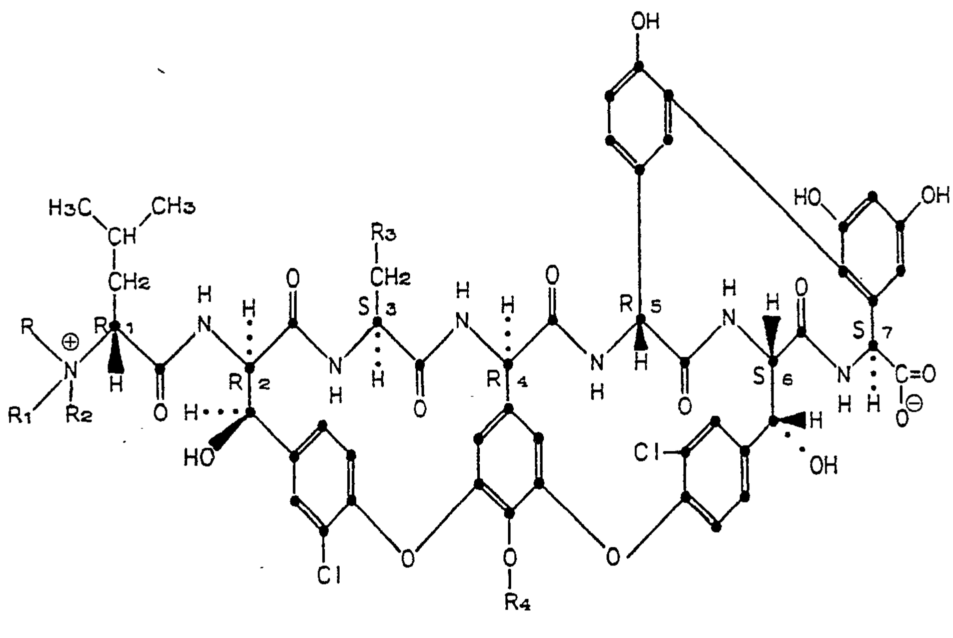

2

dans laquelle

R représente un atome d'hydrogène ou un groupe méthyle;

$R_1$ et $R_2$ représentent chacun un groupe méthyle;

$R_3$ représente $CONH_2$ ou COOH;

$R_4$ représente un groupe glucosyle ou un groupe vancosaminyl-O-glucosyle;

avec cette réserve que, lorsque $R_4$ représente un groupe glucosyle, alors R doit représenter un groupe méthyle et $R_3$ doit représenter $CONH_2$; ou

un de leurs sels pharmaceutiquement acceptable; et

en association avec moins de 10% en poids de vancomycine, ce procédé consistant à cultiver *Nocardia orientalis* NRRL 2450 ou NRRL 2452, ou un de ses variant, mutant ou recombinant producteur de M5-18260 ou de M43-05865, dans un milieu de culture contenant des sources assimilables de carbone, d'azote, et de sels inorganiques, dans des conditions de fermentation aérobie submergée.

2. Procédé selon la revendication 1, celui-ci étant suivi de la séparation du complexe hors du milieu de culture et de la mise en association avec moins de 10% en poids de vancomycine.

3. Procédé selon la revendication 2 dans lequel on isole l'antibiotique M43A, M43B, M43C ou M43D hors du complexe, tandis qu'on l'associe avec moins de 10% en poids de vancomycine.

4. Procédé selon la revendication 3, ce procédé consistant à préparer l'antibiotique M43A de structure:

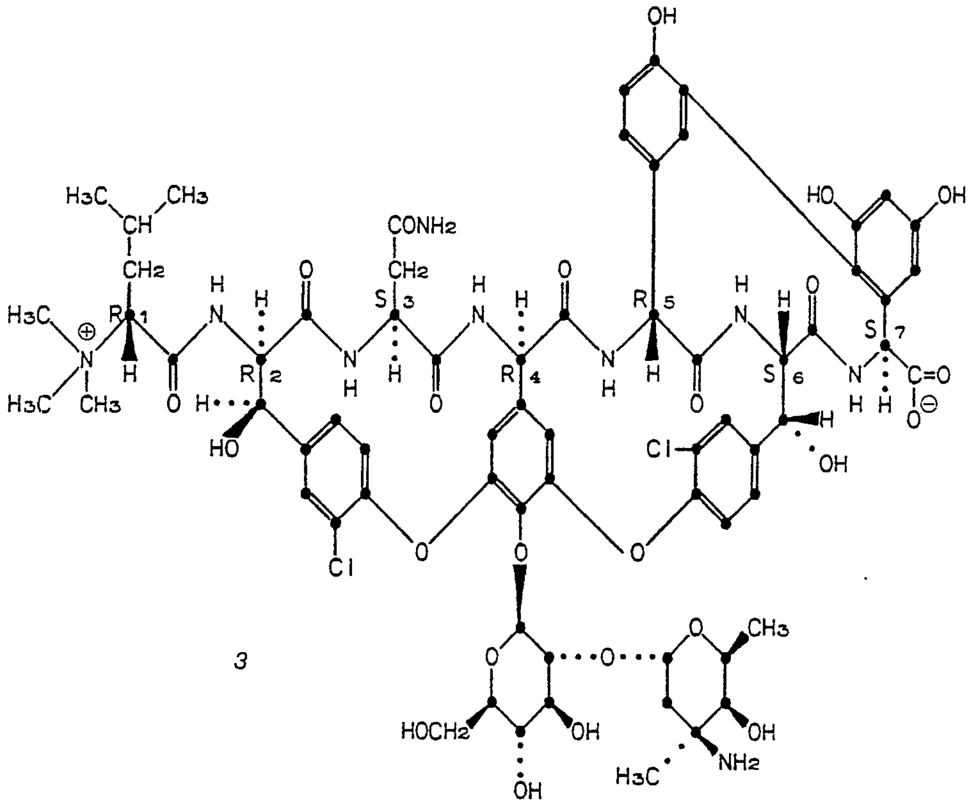

3

ou un de ses sels pharmaceutiquement acceptable, en association avec moins de 10% en poids de vancomycine.

5. Procédé selon la revendication 3, ce procédé consistant à préparer l'antibiotique M43B de structure:

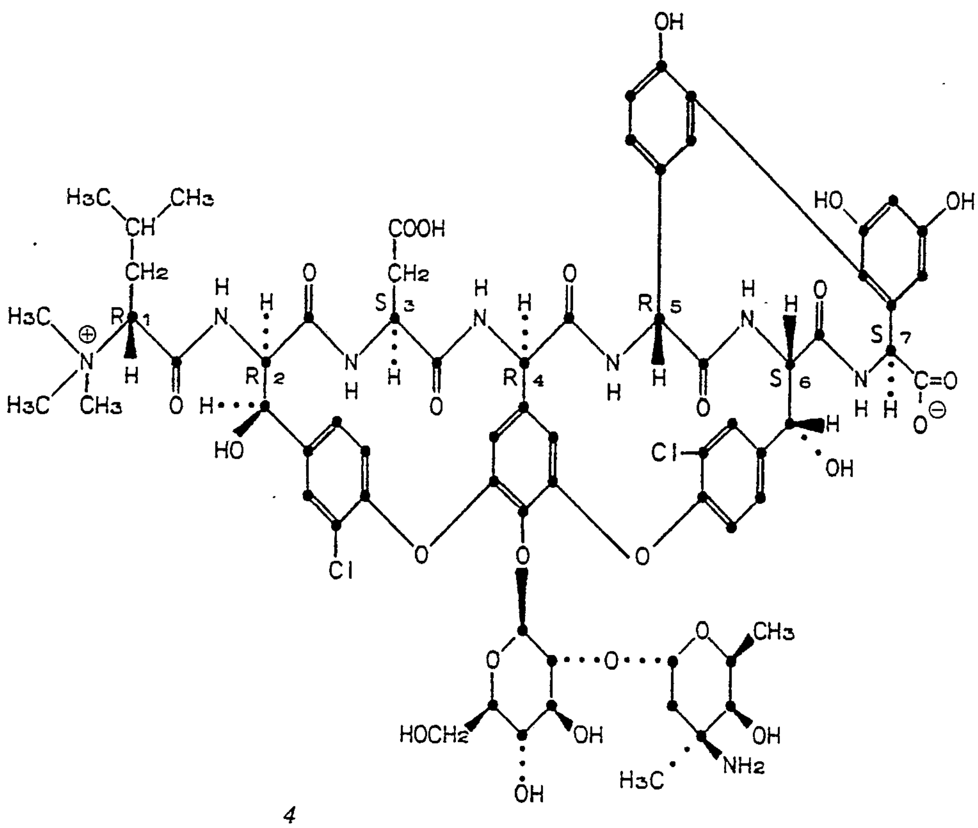

4

ou un de ses sels pharmaceutiquement acceptable, en association avec moins de 10% en poids de vancomycine.

6. Procédé selon la revendication 3, ce procédé consistant à préparer l'antibiotique M43C de structure:

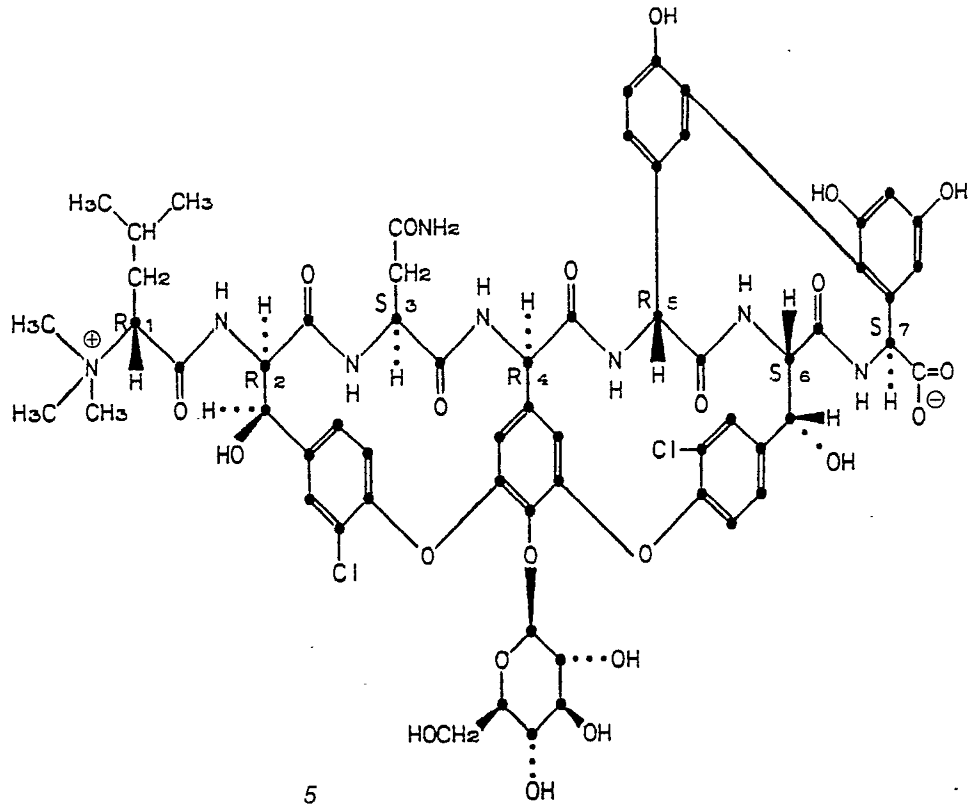

5

ou un de ses sels pharmaceutiquement acceptable, en association avec moins de 10% en poids de vancomycine.

7. Procédé selon la revendication 3, ce procédé consistant à préparer l'antibiotique M43D de structure:

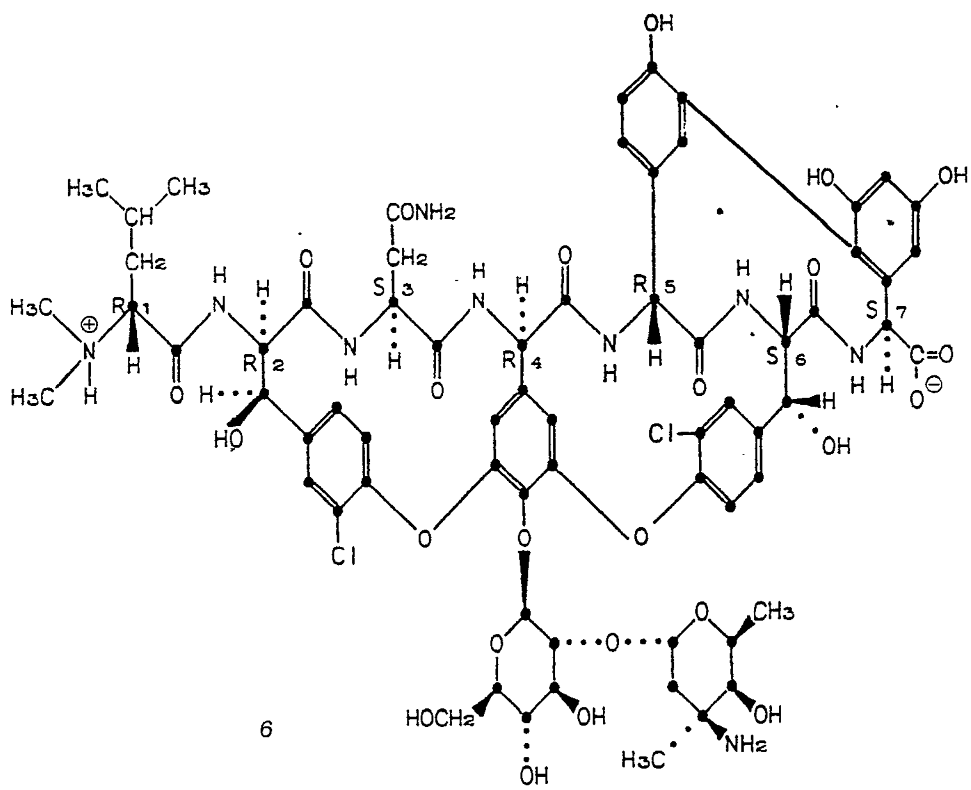

6

ou un de ses sels pharmaceutiquement acceptable, en association avec moins de 10% en poids de vancomycine.